(19) Europäisches Patentamt
European Patent Office
Office européen des brevets

(11) EP 4 712 046 A1

(12) EUROPEAN PATENT APPLICATION

(43) Date of publication:
18.03.2026 Bulletin 2026/12

(21) Application number: 25182637.6

(22) Date of filing: 13.06.2025

(51) International Patent Classification (IPC):
G06V 10/82 (2022.01)    G06N 3/0455 (2023.01)

(52) Cooperative Patent Classification (CPC):
G06V 10/82; G06N 3/045; G06N 3/0455;
G06N 3/0464; G06N 3/047; G06N 3/0475;
G06N 3/048; G06N 3/08; G06N 3/082; G06N 3/084;
G06N 3/088; G06N 3/09; G06N 3/094; G06N 3/096

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 13.09.2024 US 202463694228 P
13.09.2024 EP 24465573

(71) Applicant: Siemens Healthineers AG
91301 Forchheim (DE)
(72) Inventors:
• Serban, Alexandru Constantin
900520 Constanta (RO)
• Passerini, Tiziano
Plainsboro, NJ, 08536 (US)
• Ciusdel, Costin Florian
105100 Azuga (RO)

(74) Representative: Schwarz, Claudia
Schwarz + Kollegen
Patentanwälte
Heilmannstraße 19
81479 München (DE)

(54) TECHNIQUE FOR CONCEPT AND STYLE PRE-TRAINING FOR A PERCEPTION TASK

(57) The invention relates to a technique for pre-training a principal encoder and a concept head. A method comprises receiving, at a principal encoder (302), a medical image (502) and processing it for obtaining a principal latent representation (504), which is provided to a concept head (306) and to a style head (310) to obtain a first vector of discretized anatomical concepts and an associated further first vector of continuous styles per discretized anatomical concept in the medical image (502), respectively. An auxiliary feature decoder determines, based on the obtained first vector of discretized anatomical concepts, an auxiliary latent representation (10), based on which an auxiliary image decoder (316) performs a reconstruction (512) of the medical image (502). The principal encoder (302) and concept head (306) are pre-trained based a reconstruction loss between the received medical image (502) and the first reconstruction (512).

FIG 5

EP 4 712 046 A1

**Description**

The present invention relates to a technique for pre-training a principal encoder and a concept head, such as for performing a downstream perception task, in particular comprising a method, a computing device, a system comprising the computing device, and a computer program product.

Until recently, pretraining has primarily used self-supervised learning (SSL) techniques, which include methods such as contrastive learning [1], learning pretext tasks [2], and masked-image modelling [3]. Additionally, some approaches have used a combination of these methods to improve the pretraining process [4]. These self-supervised strategies allow models to learn from vast amounts of unlabelled data by identifying and using intrinsic patterns and relationships within the data, such as using context to reconstruct masked parts of an image.

The core idea of SSL pre-training is to develop meaningful representations from input samples, represented as a single continuous embedding vector encapsulating the content displayed in an input. These representations can be viewed as an aggregation of local concepts, their corresponding styles and their contribution on the overall meaning of the input. The nature of the representations learnt can vary depending on the specific method employed. For example, some methods encourage the representations to be similar for similar or augmented input samples, and dissimilar for samples that depict distinct concepts. Other methods aim to ensure that the representations can be accurately reconstructed from partially masked inputs or features.

The primary focus of conventional SSL techniques is on creating meaningful embeddings at the image level rather than breaking down an image into distinct concepts or styles. Usually, conventional self-supervised learning strategies rely on single-vector embeddings. Consequently, these methods fall short in identifying more granular structures, such as anatomical structures or organs, limiting their ability to capture and differentiate the specific traits and characteristics within the images.

Other methods use disentanglement representation learning, decomposing an image into separate latent variables that can identify various concepts or styles.

Regardless of the approach employed, conventional SSL methods usually aim to develop a single-vector representation of the input, which may fail to capture fine-grained concepts present in it. For example, a 2D echocardiography of the heart can be broken down into concepts such as heart chambers, valves, and walls. However, the SSL methods' single-vector representation makes it challenging to discern whether such concepts are learned during pre-training.

Moreover, similarity constraints imposed in SSL under various augmentations can cause algorithms to merge certain concepts and their associated styles. For example, two augmented views of the same input must produce similar representations. However, cropping or zooming can exclude some object parts from a view, while blurring or color jittering can alter local textures, making them different between the augmented views. This is one reason why SSL pre-trained models typically do not perform well on localized tasks, such as detecting localized pathologies, instance retrieval, or Out-of-Distribution (OOD) detection.

It is therefore an object of the present invention to provide a solution for encoding a medical image, and/or extracting its features that that is versatile and effective for various applications beyond just concept and style identification, and which can enhance the utility and performance in practical medical imaging scenarios. Alternatively or in addition, it is an object to improve interpretability, explainability, performance, robustness and/or achieve a high level of personalization, facilitating more tailored and accurate medical image characterization and interpretation when applied to downstream tasks. Further alternatively or in addition, it is an object to improve outlier detection and information retrieval capabilities.

This object is solved by a method for pre-training a principal encoder, a concept head and a style head (e.g., as for performing a downstream perception task), by a pretraining neural network system, by a downstream perception task neural network system, by a computer program (and/or computer program product), and/or by a computer-readable storage medium according to the appended independent claims. Advantageous aspects, features and embodiments are described in the dependent claims and in the following description together with advantages.

In the following, the invention will be described with respect to the claimed method first. Features, advantages, or alternative embodiments, mentioned with respect to the method can be assigned to the other claimed objects (e.g. the computer program or a device, in particular the pretraining neural network architecture, or system or a computer program product) and vice versa. In other words, the system, apparatus or device can be improved with features described or claimed in the context of the method and vice versa. In this case, the functional features of the method are embodied by structural units of the apparatus or device or system and vice versa, respectively. The method may refer to a software implementation and the device may refer to a hardware implementation (e.g. with a spatial physical structure) or a virtualization thereof. Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the respective device or apparatus claim is configured to carry out the claimed method.

As to a method aspect, a (in particular computer-implemented) method for pre-training a principal encoder and a concept head, and optionally a style head, is provided. The pre-trained principal encoder, the concept head, and the style head may be used for performing a downstream perception task. The method comprises a step of receiving, at an input layer of a principal encoder, a medical image. The method further comprises a step of processing, by the principal encoder, the medical image for obtaining a principal latent representation of the received medical image. The method further comprises a step of providing the principal latent representation to a concept head. The method further comprises a step of obtaining, by the concept head, a first vector of discretized anatomical concepts based on the principal latent representation. The method further comprises a step of providing the medical image and the obtained first vector of discretized anatomical concepts to a style head. The method further comprises a step of obtaining, by the style head, a further first vector of continuous styles per discretized anatomical conception the medical image. The method further comprises a step of determining, by an auxiliary feature decoder, a first auxiliary latent representation based on the obtained first vector of discretized anatomical concepts. The method further comprises a step of performing, by an auxiliary decoder, a first reconstruction of the medical image based on the determined first auxiliary latent representation. The method still further comprises a step of pre-training the principal encoder and the concept head (e.g., for performing a downstream perception task). The pre-training is based on optimizing a loss function comprising a reconstruction loss between the received medical image and the first reconstruction of the medical image.

The method may further comprise a step of determining, by the auxiliary feature decoder, a further first auxiliary latent representation based on the obtained first vector of discretized anatomical concepts and the further first vector of continuous styles per discretized anatomical concept. The method may still further comprise a step of performing, by the auxiliary decoder, a second reconstruction of the medical image based on the determined further first auxiliary latent representation. The pre-training may further be based on optimizing a loss function comprising a reconstruction loss between the received medical image and the second reconstruction of the medical image.

By the techniques disclosed herein, a versatility, performance, robustness, adaptability, enhanced interpretability, and/or explainability of large-scale foundation models may be improved. Alternatively or in addition, a high level of personalization, more tailored and accurate medical image characterization and interpretation may be achieved. Further alternatively or in addition, inherent outlier detection and/or information retrieval capabilities may be highly effective from the outset. Alternatively or in addition, the disclosed pre-training techniques do not require annotations, which conventionally limit the sizes of training datasets.

By the first vector of discretized anatomical concepts, and a further first vector of continuous styles (also: attributes) associated with the first vector of discretized anatomical concepts, in particular a latent representation (and/or compact representation, such as requiring less memory space than the principal latent representation) is provided that can be used for a plurality of different downstream perception tasks, improving the performance of each downstream perception task.

The principal encoder and the concept head, and optionally a style head (also: attributed head) for obtaining the further first vector of continuous styles, are pre-trained on a plurality of medical image received from a medical imaging modality. An architecture of the principal encoder, auxiliary encoder, concept head, and optionally the style head, may be selected depending on a dimensionality (in particular 2D or 3D) of the medical images acquired by means of the type of medical imaging modality, and/or depending on further properties of the medical imaging modality, such as imaging parameters (e.g., frequency, color, and/or grayscale) and/or spectra obtained (e.g. multi-spectral data or scalar data).

The principal encoder, a principal decoder, a principal encoder-decoder pair, and/or the principal latent representation may also be denoted as first encoder, first decoder, first encoder-decoder pair, and/or first latent representation, respectively. Alternatively or in addition, the auxiliary encoder, an auxiliary decoder, an auxiliary encoder-decoder pair, and/or the auxiliary latent representation may be denoted as second encoder, second decoder, second encoder-decoder pair, and/or second latent representation, respectively. Said differently, the expressions "first" and "second" need not correspond to a rank, and do not denote any ordering (e.g., of encoders one after the other) of neural network components. To the contrary, the principal encoder and the auxiliary encoder operate in parallel based on different inputs, namely an original medical image and an augmented version of the same medical image, respectively.

Augmenting the medical image may comprise cropping, zooming, blurring, color jittering, adding noise, masking, translating, rotating, spatially shifting, shearing, and/or gamma contrast changing the medical image. The blurring may comprise a Gaussian blurring.

The vectors of discretized anatomical concepts obtained based on the principal latent representation and on the auxiliary latent representation are denoted as first vector and second vector, respectively, to avoid confusion with the mathematical concept of a "principal vector". Further vectors of continuous styles are analogously denoted as further first vector and further second vector, if they are obtained based on the principal latent representation and on the auxiliary latent representation, respectively.

The further first vector of continuous styles is, according to the technique disclosed herein, specific to the discretized anatomical concept and/or to the grid location, which in turn is associated with the discretized anatomical concept. Said differently, the further first vector of continuous styles is obtained per anatomical concept and/or per grid location.

The medical image (also: input image; briefly: image) may be a two-dimensional (2D) medical image. In particular, the medical imaging modality may be ultrasound (e.g., echocardiography), radiography (also: X-ray imaging), angiography, or scintigraphy.

In case of an ultrasound image, the image region of interest may be confined to a cone, with background (and/or no ultrasound signal) received from the regions outside the cone.

In alternative embodiments, the medical image may be a three-dimensional (3D) medical image. Alternatively or in addition, the medical imaging modality may be computed tomography (CT), magnetic resonance tomography (MRT), single-photon emission computed tomography (SPECT), and/or positron emission tomography (PET).

In some examples, the medical image may comprise a combination of two medical images acquired by means of different medical imaging modalities, e.g., by a scanner combining PET-CT or PET-MRT. In such a case, the pre-training is performed by the combination of the different medical imaging modalities.

In some embodiments, the medical image may comprise a time-like dimension, such as a video stream or time-series of medical images. For example, ultrasound imaging may comprise a video acquisition.

In some embodiments, the medical image may be accompanied by text, such as a radiology report.

A network architecture may differ between the pre-training and training and/or inference phases for performing the downstream perception task. For example, during the pre-training disclosed herein, the network architecture may comprise a principal encoder-decoder pair, an auxiliary encoder-decoder pair, the concept head and the style head.

During the inference phase, and/or during a training phase for a specific downstream perception task, the network architecture may comprise the pre-trained principal encoder, the pre-trained concept head, optionally the style head, and a downstream perception task-specific head.

The principal encoder may receive the (in particular original) medical image, and/or the medical image as acquired by a medical scanner and potentially pre-processed (e.g., in case of a CT scan for reconstructing the image from raw measurement data).

The auxiliary encoder may receive an augmented version of the medical image. Augmenting the medical image may comprise a geometric transformation (such as a translation, a rotation, a spatial shift, cropping, and/or zooming), masking, adding noise, blurring, color jittering, and/or gamma contrast changing.

By augmenting the medical image, it is expected that the discrete anatomical concepts are preserved (in particular up to geometric transformation w.r.t. their location within the medical image), while the continuous styles can change.

The (in particular principal and/or auxiliary) latent representation output by the corresponding (in particular principal and/or auxiliary) encoder may also be denoted as feature representation, latent embedding (briefly also: embedding) or reduced representation. The latent representation may in particular be reduced in terms of required memory space (e.g., a number of required bytes) compared to the original medical image.

According to the technique disclosed herein, the principal encoder and the auxiliary encoder process the original medical image and the augmented version of the medical image in parallel (e.g., substantially simultaneously). Alternatively or in addition, the principal encoder and the auxiliary encoder are, according to the present disclosure, pre-trained in parallel (and/or substantially simultaneously).

The auxiliary encoder, and optionally a principal decoder and/or an auxiliary decoder, may serve in the pre-training for using reconstruction losses as part of the loss function. The principal decoder may in some cases be present in the training and/or inference phase for cross-checking and/or supervision purposes (e.g., by performing an image reconstruction by the principal encoder-decoder pair in parallel to another task, which is performed by the principal encoder, the concept head, optionally the style head and a downstream perception task head, the correct operating of the principal encoder may be ensured). The auxiliary encoder-decoder pair may in particular be absent in the inference phase.

Any of the reconstruction losses disclosed herein enables SSL, and/or does not require labels for the medical images used for the pre-training. Different reconstruction losses may be combined in the loss function, such as using the original or an augmented version of the medical image, providing the latent representation using the principal encoder or auxiliary encoder, and/or performing the reconstruction using the principal decoder or auxiliary decoder. Alternatively or in addition, when combining an encoder and a decoder from different encoder-decoder pairs, such as the principal encoder with the auxiliary decoder (or vice versa), one or more copies of the concept head and/or the style head may be used to transition from one (e.g., the principal) latent representation to another (e.g., the auxiliary) latent representation.

After the pretraining, any network component, such as the downstream perception task-specific head may be fine-tuned (and/or further trained) for the respective downstream perception task in the inference phase.

In an inference phase, a downstream perception task-specific head may receive the vector of discretized anatomical concepts and the vector of continuous styles as obtained by employing a trained version of the principal encoder, the concept head and the style head.

The discretized anatomical concepts (also: anatomical structures) may comprise organs, anatomical structures, and/or constituent parts thereof, such as a bone, an extremity / limb, a heart chamber, valve, blood pool, and/or wall (e.g., the septum wall and/or left ventricle, LV, wall). Alternatively or in addition, an anatomical concept may correspond to a - in particular semantic - segmentation class. The anatomical concepts may in particular correspond to a predetermined set of segmentation classes (e.g., each associated with an organ) and/or segmentation sub-classes (e.g., each associated with one of several constituent parts of an organ, such as the heart ventricles and heart valves).

The concept head (also: concept discretizer) may be a classification head. Alternatively or in addition, the concepts may correspond to a predefined number of classes. The classes may comprise semantic classes and/or anatomical classes, such as organs, anatomical structures, and/or constituent parts thereof. The predefined number of classes may be provided as input, in particular by a user, such as by means of a user interface (UI), in particular a graphical user interface (GUI). In an alternative embodiment, the number of concepts or classes may be automatically determined during the pre-training of the concept head.

The output of the concept head (and/or the first vector of discretized anatomical concepts) may correspond to a (e.g., 2D) grid of concept probability distributions per medical image region.

The style head may also be denoted as concept stylizer and/or attribute head.

The output of the style head (and/or the further first vector of continuous styles) may comprise low-level information related to the concept. Low-level information may comprise data characterizing the respective concept, in particular represented in the part of the medical image. Low-level information may comprise of stylistic characteristics that do not alter the shape and structure of a concept. These characteristics may comprise of texture and detail data, lightning, contrast and details, orientation and planes, or simulate the presence of artifacts from the acquisition process, such as motion artifacts.

While throughout this disclosure, one concept head and one style head are referred to, the inventive technique may make use of two or more concept heads and/or style heads. For example, the concept head and style head receiving the principal latent representation may correspond to a "principal concept head" and a "principal style head", respectively. During the pre-training phase, an "auxiliary concept head" and an "auxiliary style head" may be used. E.g., the "auxiliary concept head" and "auxiliary style head" may be updated (and/or pre-trained) at the same rate as the auxiliary encoder and the auxiliary decoder (e.g., according to an exponential moving average, EMA).

The original medical image may, at least roughly, be re-constructable from the first vector of discretized anatomical concepts. For example, semantic information (and/or pattern information) may essentially be identical.

By combining the continuous styles with each concept, a similarity between the original medical image and the reconstructed medical image may be improved.

The downstream perception task may comprise an information retrieval, a reconstruction of input data, an object classification, an object detection, (in particular semantic) segmentation, pattern recognition, disease identification, region-based instance retrieval (such as searching a database for similar samples in relation to different patients with potentially similar diagnoses or diseases), an Out-of-Distribution (OOD) detection, a classification if a valve is open or closed, and/or synthetic data generation (in particular preserving the discretized anatomical concepts, and/or varying in continuous style / attribute).

In one embodiment, the downstream perception task may be based on the first vector of discretized anatomical concepts only. In another embodiment, the downstream perception task may be based on the first vector of discretized anatomical concepts and the further first vector of continuous styles. For some tasks, such object detection, the knowledge of the discretized anatomical concepts may be sufficient. For other tasks, such as synthetic data generation, which may be used for training a further neural network, it may be beneficial to have knowledge on both the discretized anatomical concepts and the associated continuous styles.

The pre-training may be unsupervised or self-supervised (SSL). Alternatively or in addition, the pre-training may comprise performing the method steps for a plurality of medical images (in particular without annotations and/or without ground truth).

The techniques disclosed herein may pre-train the principal encoder, the concept head, and the style head, by a combination of contributions to the loss function based in particular on various medical image reconstructions and/or representations by a grid of discretized anatomical concepts, which are in some examples equipped with continuous styles per concept and/or per point on the grid.

In a variant, the method for pre-training the principal encoder and the concept head (such as for performing a downstream perception task) may comprise the steps of receiving, at an input layer of the principal encoder, a medical image, processing, by the principal encoder, the medical image for obtaining a principal latent representation of the received medical image, providing the principal latent representation to the concept head, and obtaining, by the concept head, a first vector of discretized anatomical concepts based on the principal latent representation. In this variant, the method may further comprise a step of constructing a grid of discretized anatomical concepts based on the first vector of discretized anatomical concepts. The grid may be constructed by each entry of the first vector to its associated point on a lattice covering the area or volume of the medical image. The pre-training of the principal encoder and of the concept head according to this variant may be based on optimizing a loss function comprising a concept cluster loss and/or concept prior loss of the grid of discretized anatomical concepts.

The concept cluster loss may consider that overly granular concepts are undesirable. A mean square of spatial derivatives of one-hot vectors may be minimized leading to larger concept islands. By sampling the grid of concept probability distributions, a grid of one-hot vectors may be obtained. The one-hot vector grid indices may correspond to learned matrix elements of a concept embedding, leading to a 2D concept map. The minimizing of the mean square of spatial derivates may alternatively be denoted as gradient pass-through.

The concept prior loss may in particular be applicable to ultrasound image, which comprise a cone with imaged anatomical structures and only background (and/or no ultrasound signal) outside the cone. The prior may distinguish the background and the inside of the cone at a grid-location level and/or at an image level.

In any variant, the method may further comprise a step of augmenting the medical image or receiving an augmented version of the medical image. The augmenting may be, or may have previously been, performed by an augmenting unit. E.g., in parallel to receiving the medical image at the input layer of the principal encoder, the medical image may be received at the augmenting unit. The method may further comprise a step of receiving, at an input layer of an auxiliary encoder, the augmented medical image. The method may further comprise a step of processing, by the auxiliary encoder, the augmented medical image for obtaining a second auxiliary latent representation of the augmented medical image. The method may further comprise a step of providing the second auxiliary latent representation to the concept head. The method may still further comprise a step of obtaining, by the concept head, a second vector of discretized anatomical concepts based on the second auxiliary latent representation. The pre-training may be further based on optimizing the loss function comprising a concept consistency loss between the first vector of discretized anatomical concepts and the second vector of discretized anatomical concepts.

It is noted here that, throughout the disclosure, the first and further first auxiliary latent representation are obtained from the first vector of discretized anatomical concepts and from the combination of the first vector discretized anatomical concepts and the associated further first vector of continuous styles, respectively, which are obtained based on the medical image being processed by the principal encoder. By contrast, the second auxiliary latent representation is obtained from the auxiliary encoder, to which the (usually augmented) medical image is input.

The augmenting unit may be a unit that performs geometric transformations (e.g., rotating, flipping, and/or clipping) on the medical image. The augmenting unit may alternatively or in addition perform manipulation of the medical image, such as adding noise.

According to a further variant of the (in particular computer-implemented) method for pre-training the principal encoder and the concept head (such as for performing a downstream perception task), the method comprises the steps of receiving, at the input layer of the principal encoder, a medical image, processing, by the principal encoder, the medical image for obtaining a principal latent representation of the received medical image, providing the principal latent representation to the concept head and obtaining, by the concept head, a first vector of discretized anatomical concepts based on the principal latent representation. The method according to this further variant may comprise that the medical image is augmented. The medical image may in one embodiment have been previously augmented. In another embodiment, this further variant of the method may comprise a step of augmenting the medical image, for example by an augmenting unit. The method according to this further variant comprises a step of receiving, at an input layer of an auxiliary encoder, the augmented medical image. The method further comprises a step of processing, by the auxiliary encoder, the augmented medical image for obtaining a second auxiliary latent representation of the augmented medical image. The method further comprises a step of providing the second auxiliary latent representation to the concept head. The method further comprises a step of obtaining, by the concept head, a second vector of discretized anatomical concepts based on the second auxiliary latent representation. The method further comprises a step of pre-training the principal encoder and the concept head (such as for a downstream perception task). The pre-training according to this further variant is based on optimizing a loss function comprising a concept consistency loss between the first vector of discretized anatomical concepts and the second vector of discretized anatomical concepts.

The method according to the further variant may comprise a step of constructing a grid of discretized anatomical concepts based on the first vector of discretized anatomical concepts. The pre-training may be further based on optimizing the loss function comprising a concept cluster loss and/or concept prior loss of the grid of discretized anatomical concepts.

The pre-training method according to any variant comprising the principal encoder, the concept head and the style head, and optionally a principal image decoder, auxiliary encoder, auxiliary feature decoder, and/or auxiliary image decoder, can have many variants, according to which the pre-training is performed. In any case, the pre-training may be unsupervised and/or based on SSL (and/or not require annotated training data, and/or not require training data with predetermined ground truth) using a loss function with a variety of loss contributions. In particular, the pre-training may be based on one or more reconstruction losses. To improve on the reconstruction losses and avoid issues, such as very small concepts, additional guidance through losses, like the concept clustering loss, may be used complementarily. Alternatively or in addition, the prior loss may add some information about the concept/style distributions before training, and can help to have a distribution of probabilities over the concepts and/or styles.

The style head may, according to any variant of the method, be pre-trained based on a style covariance loss. Optionally, the style covariance loss comprises a constraint on unit covariance and zero mean along a grid dimensions.

The constraints may require that row-wise, the mean is zero with standard deviation of 1 and zero correlation between rows.

The principal encoder and a principal image decoder may be comprised in a principal encoder-decoder pair. The method according to any variant may comprise a step of receiving the principal latent representation at the principal image decoder. The method may further comprise a step of outputting, by the principal image decoder, a reconstruction of the medical image. The pre-training of the principal encoder, and optionally of the principal image decoder, may be further based on optimizing the loss function comprising a reconstruction loss between the medical image and the reconstruction output by the principal image decoder.

The auxiliary encoder and the auxiliary image decoder may be comprised in an auxiliary encoder-decoder pair. The method according to any variant may comprise a step of receiving a second auxiliary latent representation (which is in particular obtained by the auxiliary encoder and based on the augmented medical image) at the auxiliary image decoder. The method may further comprise outputting, by the auxiliary image decoder, a reconstruction of the augmented medical image. The auxiliary encoder-decoder pair may be pre-trained based on a reconstruction loss between the input augmented version of the medical image and the corresponding reconstruction.

Pre-training the principal encoder may imply also pre-training the auxiliary encoder. The auxiliary encoder may be pre-trained slower, such as by constraining the auxiliary encoder to changes according to an exponential moving average (EMA). Thereby, a stability of the pre-training may be improved and/or model collapse may be avoided.

The pre-training of the principal encoder, the concept head and the style head, may be further based on minimizing a reconstruction loss between the original medical image (which is received by the principal encoder, converted into a principal latent representation, based on which the concept head and the style head determine the first vector of discretized anatomical concepts and the further first vector of continuous styles, respectively, and which may be converted into a first or further first auxiliary latent representation by the auxiliary feature decoder) and the reconstruction output by the auxiliary image decoder (which may in particular be based on the second auxiliary latent representation obtained by the auxiliary encoder based on the augmented medical image). Such a pre-training may, e.g., be based on minimizing a dissimilarity between the first or further first auxiliary latent representation and the second auxiliary latent representation.

The pre-training of the principal encoder, the concept head, and the style head may be further based on minimizing a feature reconstruction loss between latent representations. The principal encoder and the auxiliary encoder may each receive the original medical image and process it to obtain a second auxiliary latent representation and a principal latent representation, respectively. Based on the principal latent representation, a first vector of discretized anatomical concepts and a further first vector of continuous styles may be obtained, which may be jointly transformed, by a feature decoder such as the auxiliary feature decoder, into a further principal latent representation. The feature reconstruction loss may be based on comparing this further principal latent representation and the second auxiliary latent representation.

While throughout this disclosure, one auxiliary feature decoder is referred to, the inventive technique may make use of multiple (e.g., auxiliary) feature decoders, for example depending on their input being a principal latent representation or an auxiliary latent representation of a medical image.

Pre-training the principal encoder, the concept head, the style head, and optionally the principal image decoder, may comprise pre-training the auxiliary encoder-decoder pair by using the concept head and the style head, for modifying parameters and/or weights of the auxiliary encoder-decoder pair. E.g., the parameters and/or weights of the concept head and the style head may be pre-trained based on using the principal encoder (and/or the encoder-decoder pair), and those parameters and/or weights may be used for pre-training the auxiliary encoder-decoder pair.

Alternatively or in addition, pre-training the principal encoder, the concept head, the style head, and optionally the principal image decoder, may comprise using the auxiliary encoder-decoder pair for (in particular directly) modifying parameters and/or weights of the concept head and the style head, and (in particular indirectly) modifying parameters and/or weights of the principal encoder.

The auxiliary encoder-decoder pair may be learning slower, e.g., according to the EMA, than the principal encoder-decoder pair. In particular, the auxiliary encoder-decoder pair may be a slower version of the principal encoder-decoder pair (e.g., based on the same neural network architecture comprising the encoder and the image decoder).

The second auxiliary latent representation provided by the auxiliary encoder may be modified by comparing the resulting second vector with the first vector and the resulting further second vector with the further first vector. An aim may be to improve pair-wise similarity.

When obtaining the individual concepts from a grid of a 2D medical image, they can be viewed as separated from the (x, y) dimensions and concatenated on the z dimension, which is also denoted as the channel dimension. The 2D medical image can be viewed as a matrix of pixels in the space of (x, y), with the channel dimension for example being the dimension of (r, g, b) values for a coloured 2D medical image. If all concepts are available (and/or known) from (x, y), and instead of arranging them one next to each other, they can be "stacked" one on top of each other, like the pages of a book, where each page would be one identified concept. These stacked concepts may then be then passed to the style head. The dimension of the stack (z) may be typically called the channel dimension, as it takes inspiration from the (r, g, b) channels.

The discretized anatomical concepts may comprise organs, anatomical structures, and/or their constituent parts. Optionally, the continuous styles comprise low-level information in relation to the associated discretized anatomical concepts, such as texture data, tissue type data, and/or any further detailed features in relation to the concept to which the style is associated.

The downstream perception task to be performed on the medical image an information retrieval may be a reconstruction, an object classification, an object detection, a semantic segmentation, a pattern recognition, a disease identification, a region-based instance retrieval, an Out-of-Distribution (OOD) detection, a classification if a valve is open or closed, and/or synthetic data generation.

The downstream perception task may be configured for clinical decision support (CDS).

The region-based instance retrieval may comprise searching a database of medical images for similar samples. Thereby, patients with similar medical features may be found. Knowing the medical history and treatment plan for the patients with similar medical features may provide CDS.

By the OOD detection, rare cases, such as rare lesions, may be detected.

For example, by the classification if the valve is open or closed, a phase, such as a phase of the cardiac cycle, may be determined, during which the medical image was acquired.

Synthetic data generation may provide further medical images with preserved anatomical concepts, but varying styles. The synthetic data generated may, e.g., be used for training a further neural network.

A concept is attributed to a minimal size of a set of adjacent pixels or voxels.

A concept may be attributed to, or may have, a (e.g., minimal) concept size, which can be defined by a relative size of a region such as a number of pixels or voxels. A minimal concept size may be a set. The minimal concept size and/or the relative size of the region may be intrinsic to a choice of neural network components.

The smallest concept may, e.g., comprise 5 times 5 pixels for a 2D medical image.

The smallest concept may correspond to a receptive field of view, and/or may be measured in k times k (time k) grid locations for a 2D (or 3D) medical image. By requiring the receptive field of view to be larger than one, smooth pixel-level transitions between adjacent concepts are facilitated. By having a grid that is smaller than the full grid, granular region descriptors are constructed that prevent the model from exploiting non-local relations.

According to a use aspect, the principal encoder, concept head and style head pre-trained according to the method aspect may be used in combination with a downstream perception task-specific head for performing a downstream perception task.

According to a first device aspect, a pre-training neural network architecture (also: pre-training neural network system) for pre-training a principal encoder and a concept head, in particular for performing a downstream perception task, is provided. The pre-training neural network architecture comprises a principal encoder, which is configured for rreceiving, at an input layer, a medical image and is further configured for processing the medical image for obtaining a principal latent representation of the received medical image. The pre-training neural network architecture further comprises a concept head which is configured for receiving the principal latent representation and is further configured for obtaining a first vector of discretized anatomical concepts based on the principal latent representation. The pre-training neural network architecture further comprises a style head, which is configured for receiving the principal latent representation and the obtained first vector of discretized anatomical concepts to a style head and further configured for obtaining a further first vector of continuous styles per discretized anatomical concept in the medical image based on the first vector and on the principal latent representation. The pre-training neural network architecture further comprises an auxiliary feature decoder, which is configured for determining a first auxiliary latent representation based on the obtained first vector of discretized anatomical concepts. The pre-training neural network architecture further comprises an auxiliary image decoder, which is configured for performing a first reconstruction of the medical image based on the determined first auxiliary latent representation. The pre-training neural network architecture still further comprises a loss function, which is configured for pre-training the principal encoder and the concept head, wherein the pre-training is based on optimizing a loss function comprising a reconstruction loss between the received medical image and the first reconstruction of the medical image.

The pre-training neural network architecture may comprise a principal encoder-decoder pair and an auxiliary encoder-decoder pair. The auxiliary encoder-decoder pair may be a "mirror" or "slower version" (e.g., having the same architectural structure and/or layer structure) of the principal encoder-decoder pair.

The principal image decoder and/or auxiliary image decoder may in particular not have any skip connections.

The pre-training neural network architecture may be configured to perform any one of the steps, and/or comprise any one of the features, disclosed in the context of the method aspect.

According to a second device aspect, a downstream perception task neural network architecture (also: downstream perception task neural network system) is provided, which comprises a principal encoder, a concept head, a style head, and a downstream perception task-specific head. The principal encoder, the concept head and the style head have been pre-trained using the method according to any of the preceding method claims.

The pre-training neural network architecture and/or the downstream perception task neural network architecture may comprise a vision transformer. Alternatively or in addition, the pre-training (and/or training for the downstream perception task) may make use of variational inference. The pre-training neural network architecture and/or the downstream perception task neural network architecture may in particular comprise a convolutional neural network (CNN) or vision transformer (ViT). The CNN and ViT may differ in terms of architectural blocks and/or primitives.

The pre-training neural network architecture, and/or the downstream perception task neural network architecture may be embodied by a computing device. The computing device may be configured for performing the method according to the method aspect.

As to a further aspect, a computer program product is provided comprising program elements which induce a computing device to carry out the steps of the method or pre-training a principal encoder and a concept head according to the method aspect, when the program elements are loaded into a memory of the computing device.

As to a still further aspect, a computer-readable medium is provided, on which program elements are stored that can be read and executed by a computing device, in order to perform steps of the method or pre-training a principal encoder and a concept head according to the method aspect, when the program elements are executed by the computing device.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labelled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

Fig. 1            is an exemplary flow chart of a first variant of a method for pre-training a principal encoder and a concept head, e.g., for performing a downstream perception task in an inference phase;

Fig. 2            is an exemplary flow chart of a second variant of a method for pre-training a principal encoder and a concept head, e.g., for performing a downstream perception task;

Fig. 3            is an overview of the structure and architecture of a pre-training neural network for pre-training a principal encoder and a concept head, e.g., for performing a downstream perception task in an inference phase;

Fig. 4            is an exemplary overview of the structure and architecture of downstream perception task neural network, which uses the principal encoder and concept head pre-trained according to the first variant of the method in Fig. 1;

Fig. 5            shows an exemplary combination of a principal encoder-decoder pair, an auxiliary encoder-decoder pair, a concept head and a style head used in the pre-training method, such as the variants in Fig. 1 or 2;

Fig. 6            shows a further example of performing the pre-training of a principal encoder, a concept head and a style head using any one of an auxiliary encoder, an auxiliary feature decoder, an auxiliary image decoder, and a principal image decoder, with associated losses for each case;

Figs. 7A, 7B and 7C    show exemplary concept maps from randomly sampled inputs with indices of the most likely concept for each location displayed at the bottom-left of the location;

Figs. 8A, 8B and 8C    exemplarily illustrates the effect of concept swapping. In Fig. 8A, the reconstruction is based only a greedy concept map. In Fig. 8B, two modifier concepts are swapped. In Fig. 8C, big changes are induced by swapping two anatomic concepts;

Fig. 9                    illustrates region-based instance retrieval using conceptual search. In each row, a query image is shown, followed by the top three retrieval results;

Fig. 10                   illustrates the performance of the inventive technique compared to a conventional technique in terms of sensitivity and specificity;

Fig. 11                   illustrates the effect of increased noise injected in the continuous style component. In each row, the original medical image, a noise-free reconstruction and three reconstructions with increase noise are shown; and

Fig. 12                   further illustrates the effect of noisy reconstructions.

Any reference signs in the claims should not be construed as limiting the scope.

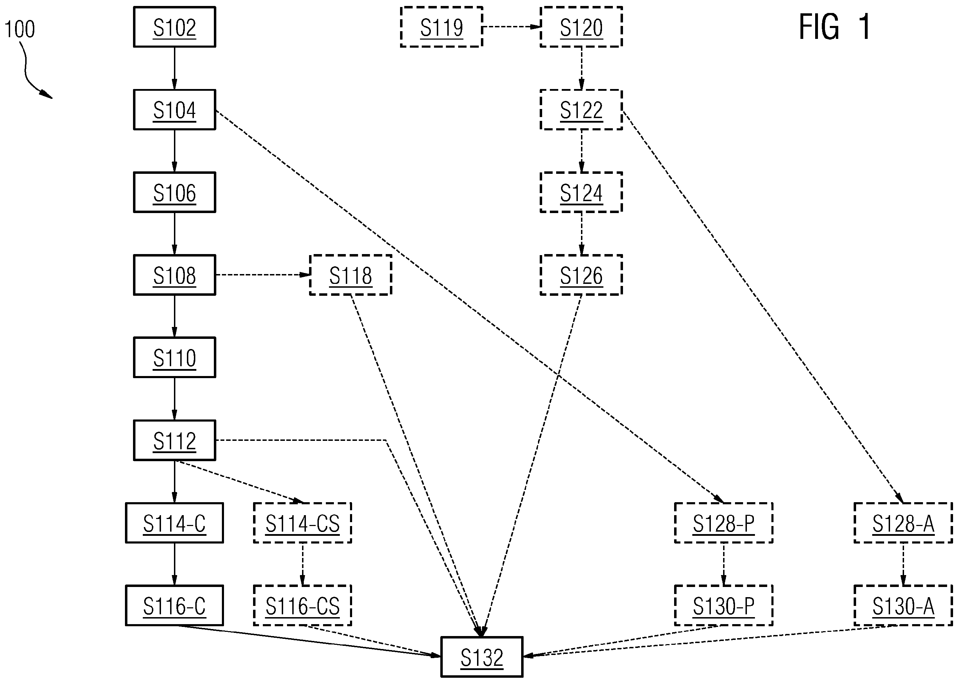

Fig. 1 schematically illustrates an exemplary flowchart for a first variant of a computer-implemented method for pre-training a principal encoder and a concept head, such as for performing a downstream perception task. The first variant of the method is generally referred to by the reference sign 100.

The method 100 comprises a step S102 of receiving, at an input layer of a principal encoder, a medical image. The method 100 further comprises a step S104 of processing, by the principal encoder, the medical image for obtaining a principal latent representation of the received S102 medical image. The method 100 further comprises a step S106 of providing the principal latent representation to a concept head. The method 100 further comprises a step S108 of obtaining, by the concept head, a first vector of discretized anatomical concepts based on the principal latent representation. The method 100 further comprises a step S110 of providing the principal latent representation and the obtained S108 first vector of discretized anatomical concepts to a style head. The method 100 further comprises a step S112 of obtaining, by the style head, a further first vector of continuous styles per discretized anatomical concept in the medical image based on the first vector of discretized anatomical concepts and on the principal latent representation.

The method 100 further comprises a step S114-C of determining, by an auxiliary feature decoder, a first auxiliary latent representation based on the obtained first vector of discretized anatomical concepts. The method 100 further comprises a step S116-C of performing, by an auxiliary image decoder, a first reconstruction of the medical image based on the determined first auxiliary latent representation. The method 100 further comprises a step S132 of pre-training the principal encoder and the concept head. The pre-training S132 is based on optimizing a loss function comprising a reconstruction loss between the received S102 medical image and the first reconstruction of the medical image.

Optionally, the method 100 comprises a step S114-CS of determining, by the auxiliary feature decoder, a further first auxiliary latent representation based on the obtained first vector of discretized anatomical concepts and the further first vector of continuous styles per discretized anatomical concept. The method 100 may further comprise a step S116-CS of performing, by the auxiliary image decoder, a second reconstruction of the medical image based on the determined further first auxiliary latent representation. The pre-training S132 may be further based on optimizing a loss function comprising a reconstruction loss between the received S102 medical image and the second reconstruction of the medical image.

By reconstructing the medical image from the discretized anatomical concepts, it can be ensured that the discretized anatomical concepts are representative of the medical image. The reconstruction may be viewed as a proxy for labels and/or enable SSL training.

The method 100 may comprise a step S118 of constructing a grid of discretized anatomical concepts based on the first vector of discretized anatomical concepts. The grid may be constructed S118 by allocating each entry of the first vector to its associated point on a lattice covering the area or volume of the medical image. The pre-training S132 may be further based on optimizing the loss function comprising a concept cluster loss and/or concept prior loss of the grid of discretized anatomical concepts.

Optionally, the method 100 comprises a step S119 of augmenting the medical image. The method 100 may further comprise a step S120 of receiving, at an input layer of an auxiliary encoder, the augmented S119 medical image. The method 100 may further comprise a step S122 of processing, by the auxiliary encoder, the augmented medical image for obtaining a second auxiliary latent representation of the augmented medical image. The method 100 may further comprise a step S124 of providing the second auxiliary latent representation to the concept head. The method 100 may still further comprise a step S126 of obtaining, by the concept head, a second vector of discretized anatomical concepts based on the second auxiliary latent representation. The pre-training S132 may be further based on optimizing the loss function comprising a concept consistency loss between the first vector of discretized anatomical concepts and the second vector of discretized anatomical concepts.

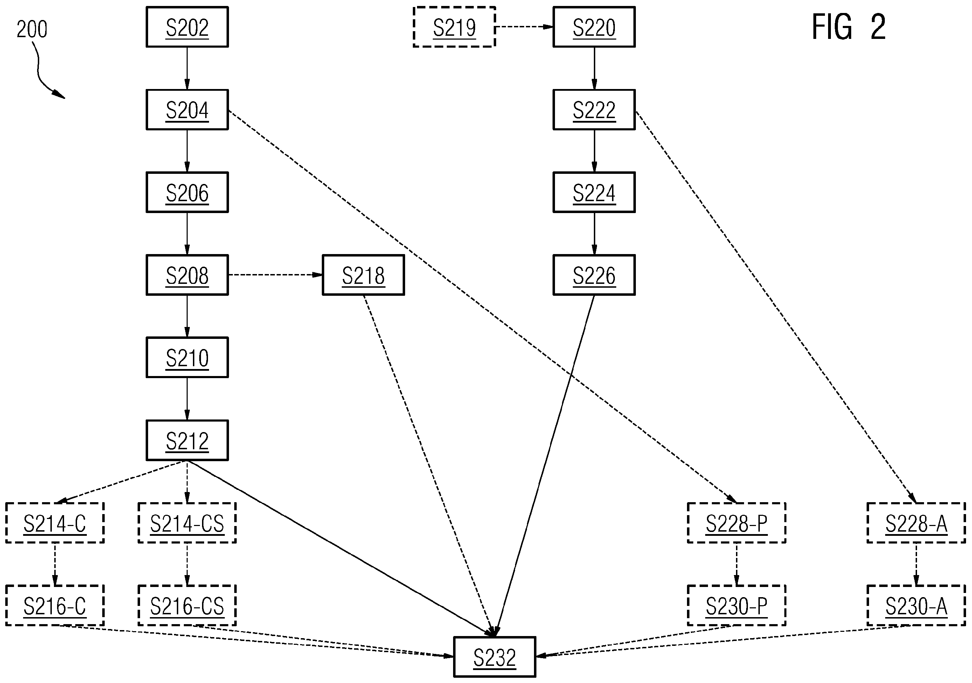

Fig. 2 schematically illustrates an exemplary flowchart for a second variant of a computer-implemented method for pre-training a principal encoder and a concept head, such as for performing a downstream perception task. The second variant of the method is generally referred to by the reference sign 200.

The method 200 starts essentially identical to the method 100. The method 200 comprises a step S202 of receiving, at an input layer of a principal encoder, a medical image. The method 200 further comprises a step S204 of processing, by the principal encoder, the medical image for obtaining a principal latent representation of the received S202 medical image. The method 200 further comprises a step S206 of providing the principal latent representation to a concept head. The method 200 further comprises a step S208 of obtaining, by the concept head, a first vector of discretized anatomical concepts based on the principal latent representation. The method 200 further comprises a step S210 of providing the principal latent representation and the obtained S208 first vector of discretized anatomical concepts to a style head. The method 200 further comprises a step S212 of obtaining, by the style head, a further first vector of continuous styles per discretized anatomical concept in the medical image based on the first vector of discretized anatomical concepts and on the principal latent representation.

Optionally, the method 200 comprises a step S219 of augmenting the same medical image, which is input to (and/or received S202 by) the input layer of the principal encoder.

The method 200 further comprises a step S220 of receiving, at an input layer of an auxiliary encoder, an augmented S219 version of the medical image. The receiving S220 of the augmented S219 medical image at the auxiliary encoder may in particular happen in parallel to the receiving S202 of the original medical image at the principal encoder. The method 200 further comprises a step S222 of processing, by the auxiliary encoder, the augmented S219 medical image for obtaining a second auxiliary latent representation of the augmented S219 medical image. The method 200 further comprises a step S224 of providing the second auxiliary latent representation to the concept head. The method 200 further comprised a step S226 of obtaining, by the concept head, a second vector of discretized anatomical concepts based on the second auxiliary latent representation. The method 200 still further comprised a step S232 of pre-training the principal encoder and the concept head. The pre-training S232 is based on optimizing a loss function comprising a concept consistency loss between the first vector of discretized anatomical concepts and the second vector of discretized anatomical concepts.

Optionally, the method 200 comprises a step S218 of constructing a grid of discretized anatomical concepts based on the first vector of discretized anatomical concepts. The pre-training S232 may be further based on optimizing the loss function comprising a concept cluster loss and/or concept prior loss of the grid of discretized anatomical concepts.

The method 200 may further comprise a step S214-C of determining, by an auxiliary feature decoder, a first auxiliary latent representation based on the obtained first vector of discretized anatomical concepts. The method 200 may further comprises a step S216-C of performing, by an auxiliary image decoder, a first reconstruction of the medical image based on the determined first auxiliary latent representation. The pre-training S232 may be further based on a reconstruction loss between the received S202 medical image and the first reconstruction S216-C of the medical image.

Further optionally, the method 200 comprises a step S214-CS of determining, by the auxiliary feature decoder, a further first auxiliary latent representation based on the obtained first vector of discretized anatomical concepts and the further first vector of continuous styles per discretized anatomical concept. The method 200 may further comprise a step S216-CS of performing, by the auxiliary image decoder, a second reconstruction of the medical image based on the determined further first auxiliary latent representation. The pre-training S232 may be further based on optimizing a reconstruction loss between the received S202 medical image and the second reconstruction S216-CS of the medical image.

The principal encoder and a principal image decoder may be comprised in a principal encoder-decoder pair. The method 100; 200 may comprise a step S128-P; S228-P of receiving the principal latent representation at the principal image decoder. The method 100; 200 may further comprise a step S130-P; S230-P of outputting, by the principal image decoder, a reconstruction of the medical image. The pre-training S132; S232 of the principal encoder, and optionally the principal image decoder, may be further based on optimizing the loss function comprising a reconstruction loss between the medical image and the reconstruction output by the principal decoder.

An auxiliary encoder and an auxiliary image decoder may be comprised in an auxiliary encoder-decoder pair. The method 100; 200 may comprise a step S128-A; S228-A of receiving, at the auxiliary image decoder, a second auxiliary latent representation output by the auxiliary encoder based on an augmented version of the medical image. The method 100; 200 may comprise a step S130-A; S230-A of outputting, by the auxiliary image decoder, a reconstruction of the augmented medical image. The auxiliary encoder-decoder pair may be pre-trained based on minimizing a reconstruction loss between the augmented medical image and the reconstruction output by the auxiliary image decoder.

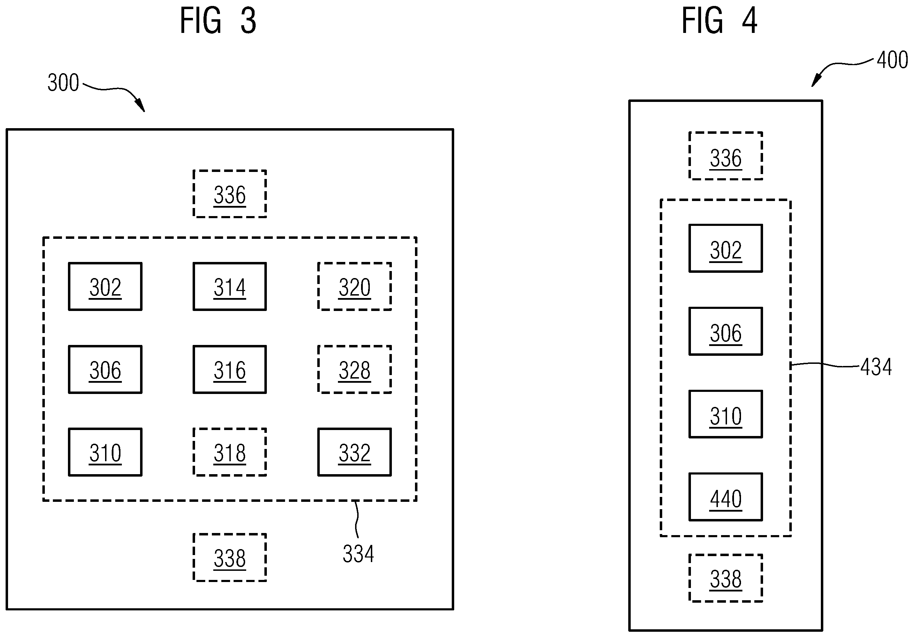

Fig. 3 schematically illustrates an exemplary pre-training neural network architecture for pre-training a principal encoder and a concept head, such as for performing a downstream perception task The pre-training neural network architecture is generally referred to by the reference sign 300.

The pre-training neural network architecture comprises a principal encoder 302, which is configured for rreceiving, at an input layer, a medical image and is further configured for processing the medical image for obtaining a principal latent representation of the received medical image. The pre-training neural network architecture further comprises a concept head 306, which is configured for receiving the principal latent representation and is further configured for obtaining a first vector of discretized anatomical concepts based on the principal latent representation. The pre-training neural network architecture further comprises a style head 310, which is configured for receiving the principal latent representation and the obtained first vector of discretized anatomical concepts to a style head and further configured for obtaining a further first vector of continuous styles per discretized anatomical concept in the medical image based on the first vector and on the principal latent representation. The pre-training neural network architecture further comprises an auxiliary feature decoder 314, which is configured for determining a first auxiliary latent representation based on the obtained first vector of discretized anatomical concepts. The pre-training neural network architecture further comprises an auxiliary image decoder 316, which is configured for performing a first reconstruction of the medical image based on the determined first auxiliary latent representation. The pre-training neural network architecture still further comprises a loss function 332 which is configured for pre-training the principal encoder and the concept head. The pre-training is based on optimizing a loss function comprising a reconstruction loss between the received medical image and the first reconstruction of the medical image.

Optionally, the pre-training neural network architecture 300 comprises an input-output (I/O) interface 336, which is configured for receiving the medical image, and optionally an augmented version of the medical image. Alternatively or in addition, the I/O interface 336 may be configured for outputting any of the reconstructions, concepts, and/or associated styles.

The pre-training neural network architecture 300 may comprise a processor 334. The processor 334 may embody any one of the principal encoder 302, the concept head 306, the style head 310, the auxiliary feature decoder 314, auxiliary image decoder 316, the loss function 332, a grid constructing head 318, an auxiliary encoder 320, and/or a principal image decoder 328.

The pre-training neural network architecture 300 may comprise a memory 338. In the memory 338, program code for executing the method 100; 200 may be stored. Alternatively or in addition, reconstructions, concepts, and/or associated styles may be stored in the memory 338.

The pre-training neural network architecture 300 may be configured for performing the method 100.

Fig. 4 schematically illustrates an exemplary downstream perception task neural network architecture for performing a downstream perception task The downstream perception task neural network architecture is generally referred to by the reference sign 400.

The downstream perception task neural network architecture comprises a principal encoder 302, a concept head 306, a style head 310, and a downstream perception task-specific head 440. The principal encoder 302, the concept head 306 and the style head 310 have been pre-trained using the pre-training method, such as according to Fig. 1 or 2.

The downstream perception task neural network architecture 400 may comprise a processor 434. The processor 434 may embody any one of the pre-trained principal encoder 302, the pre-trained concept head 306, the pre-trained style head 310, and the downstream perception task-specific head 440.

The downstream perception task neural network architecture 400 may further comprise an I/O interface 336 configured for receiving medical image, and/or a memory 338.

The inventive technique (e.g., comprising the method 100; 200, the pre-training neural network architecture 300, and/or the downstream perception task neural network architecture 400) may alternatively be denoted as pretraining foundational models to learn fine grained concepts from medical images. The technique tackles the challenge of identifying fine-grained concepts and their corresponding styles from medical images, without explicit supervision (in particular unsupervised and/or self-supervised). For example, structures like heart chambers or valves in ultrasound (US) images are identified, along with distinctive styles, such as textures. Progress in this area advances the development of large-scale foundational models, improving their versatility, robustness, and adaptability. Consequently, this leads to more precise automated medical image analysis tools for various modalities US, CT, MRT, and others.

A novel pretraining framework (e.g., comprising the method 100; 200) enables large-scale foundational models to autonomously detect fine-grained individual structures, such as organs or their constituent parts (e.g., heart chambers, valves), without explicit supervision. The framework encourages models to discover and differentiate concepts alongside unique styles that reveal specific attributes, including textures, widths, and other detailed features. Integrating the concepts with their associated styles allows for high levels of personalization, facilitating more tailored and accurate medical image characterization and interpretation. When applied to downstream perception tasks (briefly: downstream tasks), the foundational models pretrained using this approach exhibit superior performance and robustness. Moreover, they possess inherent outlier detection and information retrieval capabilities, making them highly effective from the outset, such as for fine-grained image retrieval.

Contrarily to conventional approaches, the technique disclosed herein (e.g., comprising the method 100; 200, the pre-training neural network architecture 300, and/or the downstream perception task neural network architecture 400) does not use independent representations for concepts and styles. Instead, the style is computed as a function of both the input (such as the medical image or its latent representation output by an encoder, in particular the encoder 300) and the identified concepts. This direct interconnection between concepts and styles results in superior performance, as it allows for a more integrated understanding of the concepts represented in the image.

In contrast to the conventional separate identification of concept and style, the technique disclosed herein enables the simultaneous (and/or at least partly entangled) learning of both the concept and its style. Moreover, the simultaneous (and/or at least partly entangled) learning is framed as a pretraining problem, focusing on developing embeddings that are not only adept at identifying concepts and styles but also optimized for downstream tasks. This ensures that the learned representations are versatile and effective for various applications beyond just concept and style identification. The technique disclosed herein enhances the model's utility and performance in practical medical imaging scenarios.

A core difference to conventional techniques is that the inventive technique disclosed herein inherently performs disentanglement and is more general and can be applied to multiple downstream tasks. Core disentanglement solutions may be used mainly for image generation. The inventive technique can be classified in the family of content-style (and/or concept-style) disentanglement, just where more general techniques than the ones already available are developed according to this disclosure.

Alternatively or in addition, the concept (or idea according to the inventive technique) of styles improves disentanglement. In conventional models, both concepts and styles are entangled - concepts are entangled with each other, and also with style attributes. The inventive technique enables disentanglement of both concepts and styles, which allows to apply any style to any concept. For example, a human eye may be represented as a concept that is independent of eye color, and then color may be applied through the style component.

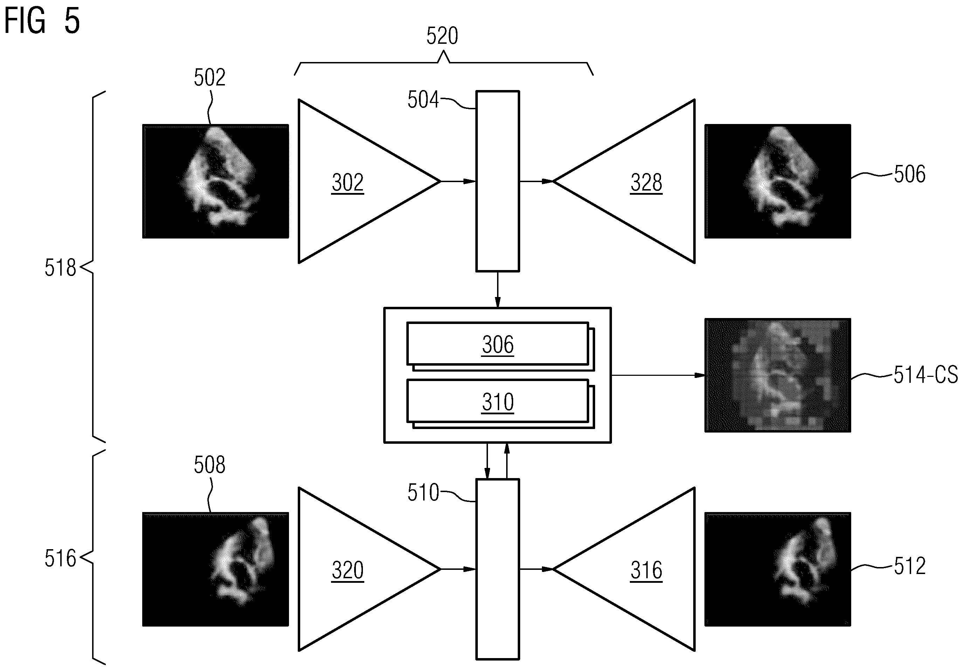

Fig. 5 provides a schematic overview of one variant of the inventive technique. This variant is also denoted as ConceptVAE: Self-Supervised Fine-Grained Concept Disentanglement from 2D Echocardiographies. It is an example of the inventive pre-training framework that can detect and disentangle fine-grained concepts from their style characteristics in a self-supervised manner.

The essence of the inventive technique lies in introducing a novel pre-training that can discretize embeddings (also: latent representations) 504; 510 into a predefined set of concepts, each associated with distinct styles. To guarantee that the learned embeddings (also: principal latent embedding 504 and/or auxiliary latent embedding 510) are meaningful and relevant for downstream perception tasks, a secondary task focused on reconstructing the input data 502, as schematically illustrated by the reconstruction 506, is incorporated.

The novelty of the technique disclosed herein can be summarized by the introduction of a novel pre-training technique that can discretize an input image into a set of predefined concepts associated with individual styles, while generating embeddings relevant for downstream tasks.

The inventive technique uses the embeddings 502; 510 of an encoder-decoder architecture, which in Fig. 5 comprises a principal encoder-decoder pair 302; 328 and an auxiliary encoder-decoder pair 320; 316, trained to reconstruct an input image 502 from its embeddings 502to discretize the content into a predefined set of concepts and associated styles. To achieve this, a discrete number of concepts are sampled from the embeddings 504, and continuous vectors of styles are associated with them. To avoid vanishing representations for the concepts, a concept consistency loss may be introduced between the concepts identified in the input, and the concept identified in an augmented version 508 of the input image that is passed through the auxiliary encoder-decoder pair 320; 316 (e.g., a copy of the network and/or principal encoder-decoder pair 302; 328) that is updated using exponential moving average (EMA).

In Fig. 5, at reference sign 518, the principal encoder-decoder pair 302; 328, the concept head 306 and style head 310 are shown to be trainable (e.g., faster) differently from the auxiliary encoder-decoder pair 302; 316 at reference sign 516. In particular the updating of the auxiliary encoder-decoder pair 302; 316 according to EMA corresponds to a slower training.

In Fig. 5, at reference sign 520 in combination with reference sign 518, it is indicated that only the principal encoder 302, the concept head 306 and the style head 310 are intended for use in a downstream perception task neural network, which will comprise a perception task-specific head.

In Fig. 5 further schematically illustrated are as inputs the original medical image 502, an augmented version 508 of the medical image, and as outputs the reconstruction 506, the reconstruction 512 and the concepts and styles 514-CS, and/or a feature reconstruction loss based on the obtained vectors of discretized anatomical concepts and continuous styles 514-CS.

The resulting foundational models can improve any medical image understanding tasks - such as object classification, detection, or segmentation - for any modality used in training.

While the explicit examples herein use as medical images ultrasound images, in particular echocardiographic images, the inventive technique can be applied to other medical imaging modalities as well, such as natural images, multi-modal settings of medical images and/or text used together for training.

The inventive technique can bring significant advantages in terms of performance by creating better representations that are aware of underlying concepts that define an image. Performance may in particular be increased for concept-level downstream tasks.

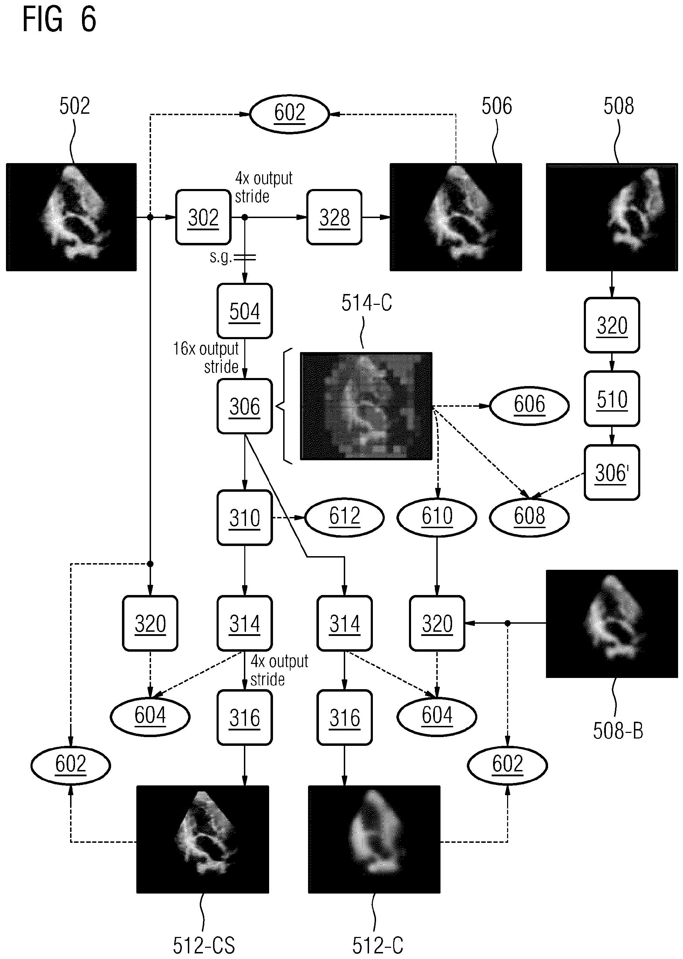

In the context of Fig. 6 to Fig. 12, further details are provided for the ConcetVAE example of the inventive technique in terms of a suite of loss terms and model architecture primitives designed to discretize input data (also: medical images) into a preset number of discretized anatomical concepts (briefly: concepts) along with their local style (also: continuous style associated with the discretized anatomical concept). ConceptVAE is validated both qualitatively and quantitatively, demonstrating its ability to detect fine-grained anatomical structures such as blood pools and septum walls from 2D cardiac echocardiographies. Quantitatively, ConceptVAE outperforms conventional self-supervised methods in tasks such as region-based instance retrieval, semantic segmentation, out-of-distribution detection, and object detection. Additionally, the generation of in-distribution synthetic data that maintains the same concepts as the training data but with distinct styles is explored, highlighting its potential for more calibrated data generation. Overall, the ConceptVAE example introduces and validates a promising new pre-training technique based on concept-style disentanglement, opening multiple avenues for developing models for medical image analysis that are more interpretable and explainable than black-box approaches.

The ability of the inventive technique to identify individual concepts that make up larger objects within input images, and capture particular traits of these concepts such as textures, can result in more expressive embeddings that can alleviate some of the weaknesses of conventional techniques, as detailed below.

The inventive pre-training technique that learns to discretize an input image into a set of fine-grained concepts, and identifies a unique set of styles for each concept is inspired by human perception, where the brain rapidly recognizes objects by first identifying essential concepts as key components and then perceiving detailed information like fine textures. The computer-implemented technique disclosed herein can be viewed as aiming to mimic this process. Using 2D cardiac echocardiographies, it is shown that the disclosed technique, which may alternatively be termed ConceptVAE, as very schematically illustrated in Fig. 5, can identify fine-grained concepts (also: discretized anatomical concepts) representing anatomical structures and regions such as heart chambers, walls or blood pools without any supervision.

The main strength of the inventive framework is the concept (content)-style disentanglement that happens natively during the pre-training procedure, a behaviour that does not occur within conventional SSL methods.

In the following, the achievement of disentanglement is demonstrated and its potential is investigated in a plurality of diverse downstream tasks (such as segmentation, object detection, retrieval, generation, outlier detection) where the combination of vectors of discretized anatomical objects and associated vectors of continuous styles per concept (also: disentangled latent space) is directly exploited. Applications in medical imaging, where aspects such as model explainability and interpretability hold great interest, can benefit from concept-style disentanglement of the latent space. Although conventional deep learning (DL) models can perform the aforementioned tasks with good performance, they lack such properties since they are black-box solutions, regardless whether pretraining was used or not in their development. Disentanglement can also be used as a tool to explore the underlying structure of data, through the explicit decomposition into observed local concepts and their style properties.

Briefly, the exemplary ConceptVAE of Fig. 5 extends the Variational Autoencoder (VAE) framework to encode a 2D input image into a latent space using a 2D grid of concept probability distributions (one $p_{ij}(c)$ for each image region, where c is a concept and $i,j$ are spatial indexes) and their associated style vectors ($s_{ij} = f(c_{ij}, x)$, where $s_{ij}$ is the style property vector of concept $c_{ij}$ that is present at location $i,j$ in input image x). It is found that even a modest number of discrete concepts and styles (e.g., 16 concepts and 8 style components) are sufficient to model 2D echocardiographies. A series of loss functions is designed that guide a neural network to detect underlying concepts from an input image and identify particular styles for each concept.

The effectiveness of the embeddings learnt is validated via ConceptVAE through distinct tasks including region-based instance retrieval, semantic segmentation, object detection, and OOD detection, demonstrating consistent improvements over more conventional SSL methods.

The inventive technique (e.g., ConceptVAE) is an SSL training framework that yields models capable of fine-grained disentangle concepts and styles from medical images. The exemplary Concept VAE model is evaluated using 2D cardiac echocardiographies, given the accessibility of datasets for pre-training and validation. Nevertheless, ConceptVAE is designed to be versatile and can potentially be applied to all 2D image modalities.

ConceptVAE is qualitatively validated, and its ability to identify concepts specialised for anatomical structures, such as blood pools or septum walls, is demonstrated.

ConceptVAE is quantitatively validated, and consistent improvements over conventional SSL methods are shown across various tasks, including instance retrieval, semantic segmentation, object detection, and OOD detection.

ConceptVAE's ability to generate data conditioned on concept semantics is assessed, and its potential to enhance robustness in dense prediction tasks is discussed.

Fig.5 presents a high-level overview of ConceptVAE. In essence, the method 100; 200 employs a VAE-like architecture to reconstruct an input from the model's embeddings (also: latent representations). It then converts the features into a set of concepts and styles via the concept head (also: concept discretizer) 306 and style head (also: concept stylizer) 310 blocks.

A self-supervised input reconstruction task is included because the model (e.g., at least the principal encoder 302, the concept head 306 and the style head 310) is trained from scratch and requires an encoder (in particular principal encoder 302) that can produce meaningful low-level embeddings. However, this task is separated (through a stop-gradient operation) from concept and style identification. Using an existing pre-trained encoder can replace this task.

To prevent feature collapse, such as unique features for all inputs or a single concept for all concept maps, as well as to improve training stability, a mirrored network 320; 316 for augmented versions of the input is used, updating it only with Exponential Moving Average (EMA)-a technique proven in SSL methods with similar aims.

Both the original 504 and augmented 520 input embeddings are transformed, discretized and styled using the concept discretizer 306 and stylizer 310 blocks. To ensure consistency in concepts between augmented versions 508 of the input 502, a specialized loss term is employed. To guide the model in learning significant concepts and styles, the original inputs 502 are reconstructed 512 from the concepts and styles using the auxiliary image decoder (also: EMA decoder) 316. A dedicated reconstruction loss term is employed to ensure that the inputs reconstructed from concepts and styles closely match the originals 502. This process encourages the model to capture and represent meaningful features of the data within the learned concepts and styles. Similarly, localised loss terms guide the model to learn diverse concepts and styles.

In the following, the architecture, the rationale behind its design, and the training procedure, including details about the selected loss function terms and optimization parameters is elaborated on.

Fig. 6 displays the detailed architecture of the exemplary ConceptVAE. A simple auto-encoder operates independently (in terms of gradients) from the rest of the model. It comprises an Encoder Stem 302 that generates features $x_{stem}$ at a 4× output stride, and an Image Decoder 328 that reconstructs 506 the original input 502. After a stop-gradient operation, an Encoder Middle 504 block applies a series of residual convolutional blocks starting from the encoder stem's features, projecting the features to concepts. The projections are used by a Concept Discretizer classification head 306, with $x_{middle}$ (corresponding to the principal latent representation 504) having a 16× output stride. For each spatial location, a Softmax activation creates a probability distribution over C concepts. Using the Gumbel-Softmax trick with hard sampling a gradient pass-through, a grid 514-C of one-hot vectors is sampled from the concept probabilities grid. This one-hot vector grid 514-C indexes a learned matrix of concept embeddings to produce a 2D concept map $x_{concept}$ (also: first vector of discretized anatomical concepts).

Subsequently, $x_{middle}$ and $x_{concept}$ are concatenated along the channel axis and passed into a Concept Stylizer 310 block. This block generates a 2D grid $x_{style}$ (also: further first vector of continuous styles) of S channels capturing the style properties of each concept. At this point, each location within the 16×-stride grid 514-C has an identified concept and an associated style vector. The channel-wise concatenation of $x_{concept}$ and $x_{style}$ constitutes the model's latent space ($x_{latent}$). Notably, $x_{concept}$ is derived from discrete embeddings, using a shared learnable embedding matrix for all input samples. In contrast, $x_{style}$ is a continuous tensor computed based on local features $x_{middle}$ and the sampled discrete concepts $x_{concept}$. Consequently, $x_{style}$ is specific to the sampled $x_{concept}$, meaning that sampling a different concept at location $i,j$ will result in a different style vector $x_{style}^{ij}$.

Fig. 6. Shows the exemplary ConceptVAE model architecture and its training setup, where the auxiliary blocks (also: EMA blocks) 320; 510; 306'; 316 (in particular EMA Encoder Stem 302, EMA encoder middle 510, EMA concept discretizer 306' and EMA mage decoder 316) represent the exponential moving average mirrors of regular blocks. Loss components 602; 604; 606; 608; 610; 612 (corresponding to image reconstruction loss 602, feature reconstruction loss 604, concept cluster loss 606, concept consistency loss 608, concept prior loss 610 and style covariance loss 612) are shown in ellipses, and "s.g." denotes stop-gradient. Solid arrows indicate tensor flows within the model, while dashed arrows represent tensors involved in loss functions.

A Feature Decoder 314 projects the latent space to reconstruct the lower 4×-stride features of the Encoder Stem 302, denoted as $x_{stem}^{rec}$. Lastly, the EMA Image Decoder 316 is employed to recover the original input image from the latent space. This reconstruction 512-C; 512-CS is core to ConceptVAE, as it guides the model to learn how to decompose an input 502 into fine-grained concepts with associated styles, and reconstruct the input 502 from concepts alone (512-C) or from concepts and associated styles (512-CS). Using the EMA Image Decoder 316 for the reconstruction ensures there is no mode collapse for the concepts or styles.

Architecturally, the Encoder Stem 302 module of Fig. 6 is designed as a simple sequence of convolutional, instance normalization, max-pooling, and Leaky ReLU stages. The final layer is a normalization layer that ensures channel-wise zero mean and unit standard deviation, helping to prevent potential feature collapse. This module 302 contains three convolutional layers with 3 × 3 kernels and strides 2, 1, 1 respectively, and one max-pooling layer with 2 × 2 kernel and stride 2, yielding a field of view size of 17 px. The Image Decoder block 316 in Fig. 6 maintains this simplicity, consisting of 2 upsampling stages based on 3 × 3 transposed convolution layers with stride 2. Regular 1 × 1 convolutions, normalization, and Leaky ReLU layers are inter-twined between the two up-sampling stages to improve the module's decoding capacity.

The Encoder Middle block at reference sign 504 employs a residual architecture. As in the Image Decoder block 328, the first layer is a Leaky ReLU activation, as the input to this block comes from the normalized convolutional output of the Encoder Stem 302. The block comprises three residual stages with 3, 5, and 5 residual layers, respectively. Each residual layer includes two sequences of normalization, Leaky ReLU, and convolution. Max-pooling and normalization layers are positioned between each residual stage. This number of layers was selected to ensure that the receptive field-of-view $x_{middle}$ exceeds the shorter dimension of the input image 502. In the exemplary case, the input image 502 has dimensions (h, w) = (256, 320), and the field of view is approximately 300 pixels. Larger or smaller architectures can be selected to model distinct input dimensions.

Equation (1) describes the operation of the concept discretizer 306. A classification head $f_{cd}$ computes the concept probability logits; Gumbel noise - $ln(- ln(u))$ is added, and a temperature ($T_{samp}$) Softmax computes the sampled concept ratios. A one-hot vector is created based on the concept with largest ratio and the pass-through technique ensures differentiability (where $sg$ is the stop-gradient operator, $I$ is the input image).

$$p(c)|\mathcal{I} = \text{Softmax}(f_{cd}\,(x\_middle\,(I)))\,,$$

$$u \sim U(0,1)\,,$$

$$p_{samp}(c) = Softmax\left(\frac{ln(p(c)\mathcal{I})-ln(-ln(u))}{T_{samp}}\right)\,, \tag{1}$$

$$y_{hard} = I_{hot}\left(argmax\left(p_{samp}(c)\right)\right),$$

$$y_{hard} = sg\left(y_{hard} - p_{samp}(c)\right) + p_{samp}(c).$$

The Concept Stylizer 310 is based on a small 3-layer sequence of convolution-Leaky ReLU-convolution layers, all with bottleneck ($1 \times 1$) kernels. Its function is to customize the selected concept at each spatial location within the $16\times$-stride grid.

The Feature Decoder 314 begins with two residual stages that process $x_{latent}$, followed by two transposed convolution stages that up-sample the grid to a $4\times$ output stride relative to the input size. These two residual stages operate on a neighborhood of $5 \times 5$ spatial locations, allowing adjacent concepts to collaborate in the reconstruction. The impact of neighborhood size on reconstruction and modeling quality is discussed further below.

Neither the Image 316; 328 nor the Feature Decoder 314 employ skip-connections that reuse internal encoder feature maps. This design is essential, as it compels the model to rely solely on its latent space, $x_{latent}$, to represent the data manifold and reconstruct the inputs.

To train ConceptVAE, a series of loss terms 602; 604; 606; 608; 610; 612 is devised inspired by classical (discrete) VAE formulations, but adapted to guide the learning process towards identifying and personalizing concepts. Two types of reconstruction losses, illustrated in Fig. 6, are employed: an image-based loss $\mathcal{L}_{img}$ at reference sign 602, which uses Mean Squared Error (MSE) over pixel values, and a feature-based loss $\mathcal{L}_{feat}$ at reference sign 604, which uses MSE over low-level feature tensors. The simple auto-encoder 302 is trained using $\mathcal{L}_{img}$ between the original input image $\mathcal{I}_{orig}$ and the reconstructed image based on the $4\times$-stride feature map. The EMA version 320 of the Encoder Stem is used to compute the target for the tensor produced by the Feature Decoder block 314, while the EMA Decoder image 316 is used to compute the reconstructed image 512-C; 512-CS from $x_{latent}$. The use of both pixel- and feature-level reconstruction losses 602; 604 has been previously employed in VAE/GAN setups, to boost both training stability and image generation fidelity.

The feature decoder 314 takes both $x_{concept}$ and $x_{style}$ as inputs. While $x_{concept}$ is generated by sampling from a discrete concept codebook, $x_{style}$ is computed directly as a (continuous) function of $x_{middle}$ and $x_{concept}$. Consequently, the network could potentially exploit this setup by minimizing the influence of $x_{concept}$ and relying more heavily on the more direct path of $x_{style}$, effectively reducing its operation to that of a simple auto-encoder. In this scenario, $x_{concept}$ would loose its semantic significance, and $x_{style}$ would function as a rich bottleneck representation rather than a style characteristic of a concept. To address this undesired behaviour, an image/feature reconstruction is performed where the style components of $x_{latent}$ are explicitly zeroed out. The EMA image Decoder 316 is reused to obtain a reconstructed version of the input image, relying solely on $x_{concept}$, without the style component $x_{style}$, at reference sign 512-C. The target of this reconstruction 512-C is a blurred version 508-B of the input image 502, with blurring serving as an approximation for removing fine details and textures, thereby partially eliminating the notion of style. Both pixel- and feature-based losses 602; 604 are employed to evaluate the reconstruction 512-C quality when using only the spatial distribution of concepts. This approach guides the Feature Decoder block 314 to focus on the concept component of $x_{latent}$ and also encourages the Encoder Middle 504 to learn to detect relevant concepts within input images 502.

Another key aspect of concept detection is its invariance to specific styles. This means that two different (augmented) views 508 of the same medical image should produce the same concept maps 514-C, despite variations in their visual appearances. Pixel-level and texture differences should be captured by $x_{style}$, while more complex anatomical structures should be encoded in $x_{concept}$. To guide this behavior during training, a Concept consistency loss 608 is introduced. The Concept Discretizer block 306 first computes a grid 514-C of concept probabilities, from which it generates a spatial grid of sampled concept indices. Following this, the concept maps 514-C from augmented views 508 should be equivalent, even if the augmentations involve translations, rotations, or other spatial shifts (here, the expression "equivalent" is used instead of "identical" because augmentations like translations, rotations, and shearing can spatially shift the placement of concepts within the image 502; 508. Nevertheless, the correspondences between the initial and shifted locations are known, and they can be used to enforce similarity between $p(c)|^{\mathcal{J}_{orig}}$ and $p(c)|^{\mathcal{J}_{augm}}$).

The EMA Encoder Stem 320, EMA Encoder Middle 510, and the EMA Concept Discretizer 306' are used to compute the target probability distributions $p_{ema}(c)$ for the concept consistency loss: $\mathcal{L}_{cc}$ = $-p_{ema}(c)$ $ln$ p(c). The EMA concept probability map $p_{ema}(c)$ is computed on an augmented view 508 of the initial input image 502 which incorporates transformations such as rotations, translations, shearings, zooming, gamma contrast changing and Gaussian blurring. Since these operations can alter positions spatial mapping between $p(c)$ and $p_{ema}(c)$ must be accounted for. To simplify this and avoid optimization noise due to imperfect mapping, each augmentation procedure selects a random location uniformly, and all image operations are performed relative to this point. The result includes a tuple of the augmented input image $\mathcal{J}_{augm}$, an initial location $l_{ij}$, and the equivalent location $l_{\hat{i}\hat{j}}$ after all operations. In this implementation of $\mathcal{L}_{cc}$, only the grid positions of the spatial locations $l_{ij}$, and $l_{\hat{i}\hat{j}}$ from $p(c)$ and $p_{ema}(c)$, respectively, were indexed. Therefore, only one pair of grid locations (containing the concept probability distributions) is used per each sample inside a training batch. The EMA blocks 302; 510; 306' are used instead of the model blocks to prevent feedback loops that could lead to collapsing concept probabilities (e.g., always detecting the same concept).

An additional constraint $\mathcal{L}_{style}$ was imposed on $x_{style}$ to ensure that it has unit covariance and zero mean along the channel (style) dimension, as illustrated at reference sign 612 in Fig. 6. Specifically, when $x_{style}$ is flattened across batches (B), height (H) and width (W), it forms a matrix of shape (S, BHW). This matrix must have a row-wise mean of 0, a row-wise standard deviation of 1, and zero correlation between rows. This constraint ensures that $x_{style}$ has independent components with a known range of values, discussed further below in detail.

To control the deviation of $p(c)|\mathcal{J}$ from $p_0(c)$, two priors are used. Without enforcing these priors during training, the entropy of $p_{ij}(c)$ would be minimized, cancelling the effect of concept sampling and reducing the model's operation to a deterministic auto- encoder. Consequently, the concept probability grid $p(c)|\mathcal{J}$ would lose much of its semantic significance, reverting to a regular discrete latent variable instead of encoding high-level semantics into a fixed set of concept probabilities. This, in turn, would constrain the functionality of the concept consistency loss 608. Two types of priors are employed: at the grid-location level and at image level. Since echocardiographies are modelled, these images typically feature an ultrasound cone centered within a surrounding black background. The grid-location level prior is computed as follows: for grid locations inside the ultrasound cone, the prior is a uniform distribution over the last $C$ - 1 concepts, with the first concept having zero mass (as w the first concept is always designated to model the background). For grid locations outside the cone, the prior assigns all probability mass to the first concept.

The KL-divergence $\mathcal{D}_{KL}$ $(p(c)|\mathcal{J}$ II $p_0(c))$ is computed at all grid locations and averaged across the (B,H, W) dimensions. For the image-level prior loss it is assumed that only the first concept should be detected outside the cone, with a uniform spread of concepts inside the cone across all samples in the current batch. Therefore, the concept probability vectors of all grid locations inside and outside the echo cones are averaged across all samples in the batch to obtain two image-level concept prevalence vectors: $d_{cone}(c)$ for the cone region and $d_{bg}(c)$ for the background.

The KL-divergence loss with the same priors is used for these concept prevalence vectors. Equation (2) formalizes the final prior loss $\mathcal{L}_{prior}$, indicated at reference sign 610 in Fig. 6, where $1_c(b, i, j)$ is an indicator function that equals 1 if location $i,j$ in sample b of the current batch pertains to an ultrasound cone. $N_{cone}$ and $N_{bg}$ are the total numbers of cone and background grid locations inside current batch, respectively,

$$\mathcal{L}_{prior1} = \sum_{b,i,j} \frac{\alpha_1}{N_{cone}} D_{KL}\left(p_{bij}(c) \mid \mathcal{I} \mid\mid p_0^{cone}(c)\right) \cdot 1_c(b,i,j)$$

$$+ \frac{\alpha_2}{N_{bg}} D_{KL}\left(p_{bij}(c) \mid \mathcal{I} \mid\mid p_0^{bg}(c)\right) \cdot \left(1 - 1_c(b,i,j)\right)$$

$$d_{cone}(c) = \frac{1}{N_{cone}} \sum_{b,i,j} (p_{bij}(c) \mid \mathcal{I}) \cdot 1_c(b,i,j)$$

$$d_{bg}(c) = \frac{1}{N_{bg}} \sum_{b,i,j} (p_{bij}(c) \mid \mathcal{I}) \cdot \left(1 - 1_c(b,i,j)\right)$$

$$\mathcal{L}_{prior2} = \alpha_3 \cdot D_{KL}\left(d_{cone}(c) \mid p_0^{cone}(c)\right) + \alpha_4 \cdot D_{KL}\left(d_{bg}(c) \mid p_0^{bg}(c)\right)$$

$$\mathcal{L}_{prior} = \mathcal{L}_{prior1} + \mathcal{L}_{prior2} . \tag{2}$$

To discourage overly granular concept maps, where sampled concepts change frequently between adjacent grid location, a Concept cluster loss $\mathcal{L}_{cluster}$, at reference sign 606 in Fig. 6 is used. Overly granular concepts are undesirable because it is desirable for concepts to represent larger anatomical structures spanning multiple grid locations rather than smaller, granular pixel patterns. To enforce it, the one-hot vectors produced by the Concept Discretizer block 306 are used. Spatial derivatives are computed between adjacent one-hot vectors along the width and height dimensions. If two adjacent locations share the same sampled concept, their one-hot vectors are identical, resulting in a null spatial derivative. Otherwise, the sampled concepts differ, leading to different one-hot vectors and a nonzero spatial derivative. By minimizing the mean square of the spatial derivative, the number of spatial transitions between sampled concepts is reduced, thereby creating larger concept "islands". The mean is taken only over grid-locations pertaining to ultrasound cones.

The final loss function is a weighted sum of the described sub-losses, as shown in Equation (3). Here, $f_{dec}(x)$ denotes the feature computed by the Feature Decoder block 314 based on its input $x$, and $I_{rec}([x_{concept}, x_{style}])$ represents the reconstructed image based on latent space components $x_{concept}$ and $x_{style}$.

$$\mathcal{L} = \beta_1 \mathcal{L}_{img}\left(\mathcal{I}_{rec}(x_{stem}), \mathcal{I}\right) + \beta_2 \mathcal{L}_{img}\left(\mathcal{I}_{rec}([x_{concept}, x_{style}]), \mathcal{I}\right) +$$
$$\beta_3 \mathcal{L}_{img}\left(\mathcal{I}_{rec}([x_{concept}, x_{style} := 0]), \mathcal{I}_{blurred}\right) +$$
$$\beta_4 \mathcal{L}_{feat}\left(f_{dec}([x_{concept}, x_{style}]), f_{stem}(\mathcal{I})\right) + \beta_5 \mathcal{L}_{feat}\left(f_{dec}([x_{concept}, x_{style} :=$$
$$0]), f_{stem}(\mathcal{I}_{blurred})\right) + \beta_6 \mathcal{L}_{style}(x_{style}) + \beta_7 \mathcal{L}_{cc}\left(p(c) \mid \mathcal{I}, p_{ema}(c) \mid \mathcal{I}_{augm}\right) +$$
$$\beta_8 \mathcal{L}_{prior}(p(c) \mid \mathcal{I}) + \beta_9 \mathcal{L}_{cluster}(x_{concept}) . \tag{3}$$

To pre-train ConceptVAE, 72,500 frames extracted from 7500 echocardiography video acquisitions were used. The dataset consisted exclusively of 2D B-mode echocardiographies featuring apical or short-axis views. The AdamW optimizer was used with a constant learning rate of $10^{-4}$, a batch size of 64 images, and a weight decay of $5 \times 10^{-3}$. During training, random image augmentations were applied using the following transformations: rotation, translation, shearing, zooming, gamma contrast adjustment, and Gaussian blurring. Pre-training is performed until convergence, which is equivalent to the loss function no longer varying significantly.

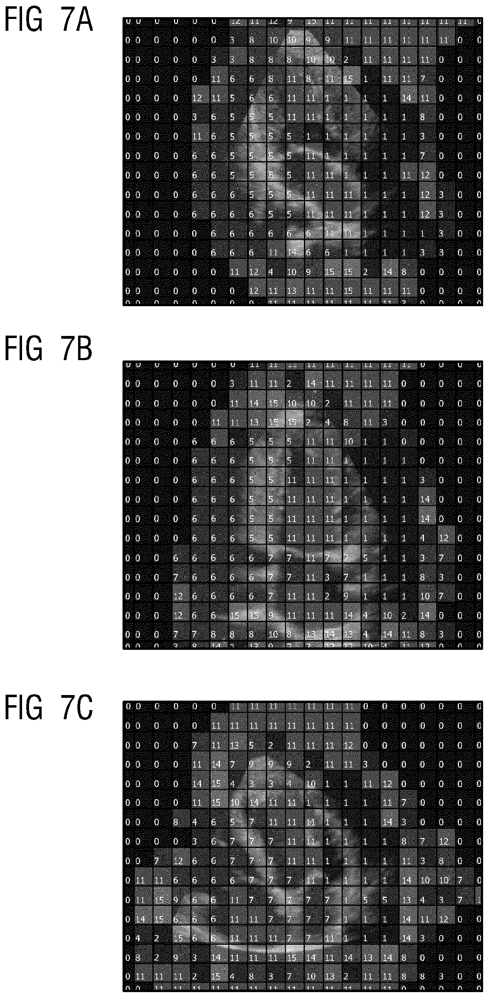

Upon convergence, the pre-trained model can be qualitatively analyzed by examining the inferred concept probability maps for test images. A straightforward method to implement this involves selecting the most likely concept at each grid location ($c_{ij} = arg\, max\, p_{ij}(c)$) and overlaying the up-sampled concept indices grid onto the initial input images, as illustrated in Figs. 7A, 7B and 7C.

Figs. 7A, 7B and 7C show concept maps for three randomly sampled inputs. The 16×-stride concept grid is up-sampled to the original image size. The indices of the most likely concept for each grid location are displayed at the bottom-left of each location. The grid may be color-coded according to concept indices for better visualization.

The probability of the most likely concept $p_{ij}(c) = max\ p(c)$ at each location $i, j$ can be incorporated in the visualizations.

By examining a random selection of samples illustrated in Figs. 7A, 7B and 7C, the following initial observations cane made:

The prior constraint 610, which requires regions outside the cone to be modeled solely by the first concept (i.e., the background concept at index 0) is generally respected.

Exceptions occur at grid locations in the cone's proximity, particularly at the boundaries between the cone and the background. As these are transition regions, they are not particularly concerning, since the model's confidence is expected to be low for such regions.

Certain concepts are specialized for specific anatomical structures. For example, concept $c_{11}$ models blood pools within the cone, concept $c_1$ represents the Left Ventricle (LV) free wall on the right hand size of the cone, concepts $c_5$ and $c_7$ correspond to septum walls, and concept $c_6$ covers the right-heart side of the cone, among others.

Certain concepts, such as e.g., $c_{13}$ and $c_{14}$ appear more isolated and spanning a single grid location. By qualitatively assessing multiple input samples, it is hypothesized that these concepts encode information about the local anatomical shapes of nearby larger concept islands. It appears that these concepts have larger confidence assigned to them than the average confidence inside larger concept islands.

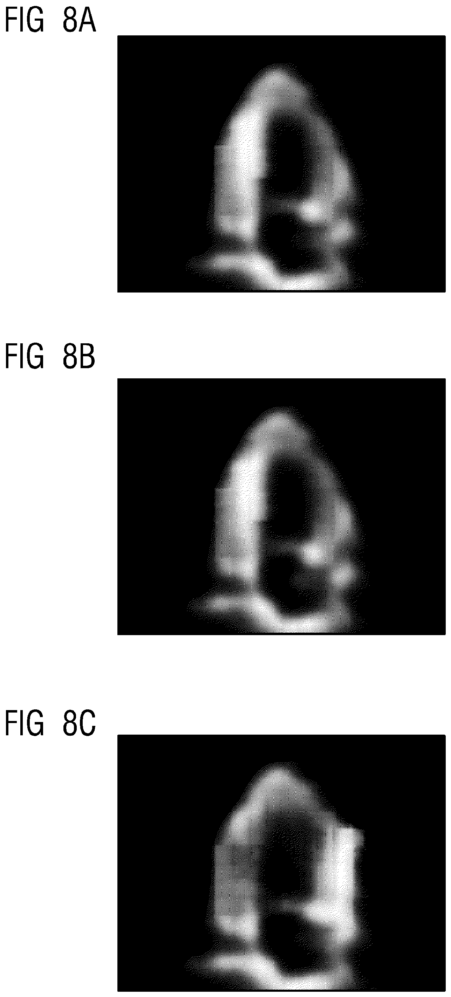

It is further hypothesized that these concepts emphasize important variations in larger concept islands. They are termed modifier concepts in this disclosure. To qualitatively evaluate the impact of modifier concepts, the greedy concept map of Fig. 7B is modified in two ways, by swapping 2 modifier and 2 normal concepts: first, (i) the modifier concepts $c_{13}$ and $c_{14}$ are swapped, and the image is reconstructed without any style component ($x_{style} := 0$); and (ii) starting from the greedy map, concepts $c_5$ and $c_1$ are now swapped, and the image is reconstructed in the same manner (with $x_{style} := 0$). The effects are illustrated in Figs. 8A, 8B and 8C: in the former case only minor shape modifications are observed around the grid locations where concept swaps were done. In the latter case, the effect is more significant, as it appears that the LV free wall changed place with the septum.

Figs. 8A, 8B and 8C illustrate the effect of concept swapping. The image in Fig. 8A is the reconstruction based only on the greedy concept map (with $x_{style} := 0$). The reconstruction in Fig. 8B illustrates the effect of swapping two modifier concepts, while the reconstruction in Fig. 8C illustrates big changes induced by swapping two anatomy-specific concepts.

While modifier concepts seem to function primarily in a styling role, it is important to note that the Feature Decoder block 314 processes k × k regions of adjacent concept locations to reconstruct the low-level image features $x_{stem}$. This means that neighboring concepts cooperate to form larger and more complex anatomical structures. Modifier concepts are not devoid of semantic meaning, as experiments showed that replacing a specialized anatomical concept like $c_1$ with a modifier concept still yields similar reconstructions, albeit with slight alterations in shape and/or region brightness patterns. Additionally, although reconstructing images based solely on $x_{concept}$ may produce rough outlines of echocardiographies, suggesting that concepts only encode basic brightness blobs, it is below shown that the concept probability grid contains rich semantics that can be used in tasks such as instance retrieval.

The region size k influences the operation and semantics of concepts. In the extreme case of k = 1, there is no concept cooperation and to match $I_{rec}([x_{concept}, x_{style} := 0])$ with $I_{blurred}$, concepts may be incentivised to encode blurred pixel patterns instead of semantic content. At the other extreme, where k equals the grid size, each grid location has a full receptive field of view, meaning it can observe the concepts from all other grid locations, regardless of distances (similar to a self-attention layer). This can be undesirable because the model may rely on non-local relations between concept placements instead of embedding semantic content within each concept. It would also hinder the extraction of local region descriptors, making it impossible to describe the content of an image crop without retaining the entire concept grid. Consequently, tasks such as region-based instance retrieval would be challenging, as it would not be clear how to construct descriptors focused on specific image regions. k = 5 was employed, meaning the receptive field of view before the up-sampling layers inside the Feature Decoder block 314 is 5 × 5 grid locations of $x_{latent}$. The rationale is that k should be large enough to allow $I_{rec}([x_{concept}, x_{style} := 0])$ to have smooth pixel-level transitions between adjacent concepts and thus be close to $I_{blurred}$d, but small enough to enable the construction of granular region descriptors and prevent the model from exploiting non-local relations.

To assess the representation power of the model's latent space quantitatively, its suitability as a general pre-training technique, and the extent of content-style disentanglement, a linear evaluation protocol tailored to SSL on several distinct tasks is employed. For comparison, a baseline model trained with Vicreg Bardes et al. is used, featuring a ResNet50 encoder and a lightweight RefineNet decoder for dense tasks. This model was pre-trained using the same dataset and configuration (e.g., image sizes) as ConceptVAE. For all following evaluation tasks, the output of the second to last ResNet stage was used as the baseline latent space (as it has the same output stride as our proposed model).

The linear evaluation protocol involved freezing the backbone and training only a linear layer on top of the frozen embeddings for specific tasks ranging from object detection to semantic segmentation or OOD detection, as detailed in the following sections.

A first downstream perception task (also: downstream task), region-based instance retrieval, involves searching a database of images for similar samples using only localized descriptors, such as pathologies or anomalies. These methods can aid in clinical diagnosis, medical research, trainee education, and support other tasks by quickly identifying patients with similar anomalies, even when a diagnosis is not yet established. SSL methods are the most prevalent and effective, using the embeddings of a pre-trained model to cluster images and retrieve those most similar to a query image using nearest neighbors search.

To use ConceptVAE for this task, image region descriptors are generated by concatenating the $5 \times 5$ concept probability vectors from a $5 \times 5$ sub-grid centered around a selected query point. The sub-grid provides context for the query point.

Using an input image of size (256, 320), the concept grid has an output stride of 16, resulting in a size of (16, 20) concepts. From each test image, an array of (14, 18) key points (i.e., all points with a complete $5 \times 5$ neighborhood) is extracted. Since the model was trained with 16 concepts and the descriptor uses a $5 \times 5$ grid, each descriptor is a vector of size 400. For the baseline model, a similar searching mechanism was used, but the region descriptor was the feature vector of a $1 \times 1$ feature map grid location. A single grid location is sufficient for this model, since its feature representation is computed in a continuous manner, without discrete variables, with a sufficiently large field of view.

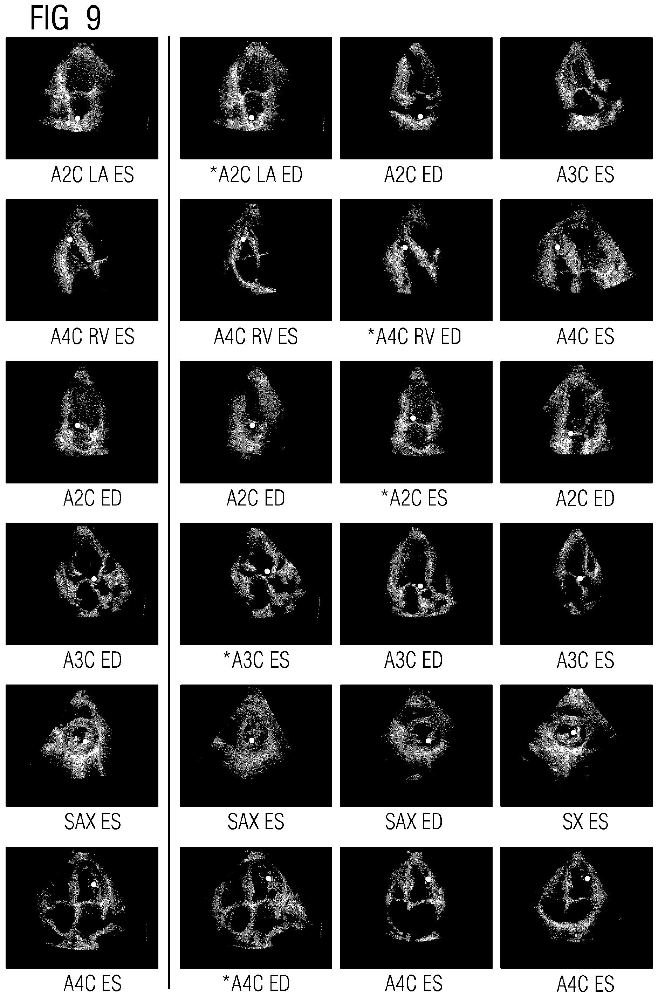

For instance retrieval, nearest-neighbor matching based on the Euclidean distance between descriptors can be employed. Initially, a qualitative analysis was conducted by randomly sampling images from the test set and manually selecting specific query points to analyze the results. The descriptors corresponding to these selected query points were then used to search the database and retrieve samples with regions similar to the query points. Fig. 9 showcases six randomly sampled examples, which illustrate that the retrieved image regions align well with the query semantics. For example, the retrieved regions share the same cardiac chamber and view as the query points. Moreover, the anatomical structures around the matched locations are visually similar to those in the query points.

Fig. 9. Illustrates region-based instance retrieval using conceptual search. The leftmost column displays query images, while the last three columns show the top-3 kNN retrieval results. Dots indicate the centers of the query and matched descriptor regions. Below each image, the view and cardiac phase are displayed. Matches marked with an asterisk (*) are from the same acquisition as the query image, but from a different cardiac phase.

For the retrieval task, the search is based solely on the concept descriptors. This approach ensures that the retrieval process focuses on the semantic content rather than stylistic variations.

To quantitatively analyze this task, an independent test set of 450 images is used, totaling 113,400 region descriptors (14 · 18 · 450). Performing nearest neighbor search on this space is very fast. The set includes four echocardiographic views (apical 2-, 3-, and 4-chamber views, and a short-axis view), with frames captured at end-diastole (ED) and end-systole (ES). For the apical views, LV contour annotations were available, from which five key landmark points were extracted: left and right annulus, apex, mid-septum, and mid- free-wall. These annotations were exploited to setup a retrieval tasks for these landmark points. In total, there were 150 ED apical frames, each with five locations used as query points. The search pool consisted of all 225 ES frames from all views, including the short-axis view. A retrieval is considered a match if it corresponds to the ES image of the ED query and if the retrieved location is adjacent to the annotated landmark point.

Results are presented in Table 1, which shows the Mean Average Precision (mAP) metrics for both models, computed using the top-5 search results. It is observed that ConceptVAE demonstrates more than double the performance of the baseline without any retraining, revealing two important observations about ConceptVAE:

The concept probability grid indeed encodes semantic content and thus $x_{concept}$ functions as a spatial arrangement of concepts, which for ConceptVAE are defined as composable higher-level discrete features.

ConceptVAE shows promising results for zero-shot instance retrieval based on local- region queries, unlike more conventional approaches that operate at the image level and need additional fine-tuning.

Table 1: Region-based instance retrieval mAP metric values

| Landmark | Model | |
| --- | --- | --- |
| | ConceptVAE | baseline |
| left annulus | 0.418 | 0.148 |
| mid-septum | 0.281 | 0.098 |
| apex | 0.518 | 0.345 |
| mid-free-wall | 0.263 | 0.094 |
| right annulus | 0.371 | 0.128 |
| average | 0.370 | 0.163 |

A second downstream task employed is semantic segmentation, where features from the pre-trained models are projected to match a down-sampled ground-truth mask. For this task, five labels are used corresponding to heart chambers: left and right ventricles and atria in apical views (A2C, A3C, and A4C views) and the left ventricle in the short-axis (SAX) view.

Starting with frozen model latent codes, a linear 2D convolutional kernel is fitted to predict low-resolution (stride $16\times$) segmentation maps. Channel-wise softmax activation is applied on top of the predicted linear logits, as shown in Equation (4). Here, $p_{ij}(s)$ represents the probability that location $i,j$ to contain chamber $s$, $x_{input}$ is the frozen latent feature map, and $W_k$ and $w_b$ are the kernel weight matrix and bias vector, respectively, and containing 6 rows for the 5 prediction targets and one background channel,

$$p_{ij}(s) = \mathsf{Softmax}\left(W_k \cdot x^{ij}_{input} + w_b\right). \tag{4}$$

The ground-truth was obtained by down-sampling the full-scale chamber masks using the area interpolation method. Training was performed on an independent set consisting of 5000 training examples, and the outcomes were tested using an independent test set of 500 samples. The Dice loss was employed as in Equation (5), where $p_{ij}$ and $t_{ij}$ are the predicted and target chamber presence probabilities at location $i, j,$ respectively,

$$\mathcal{L}_{Dice} = 1 - \frac{2\sum_{i,j} p_{ij} t_{ij}}{\sum_{i,j} p_{ij}^2 + t_{ij}^2}. \tag{5}$$

Three scenarios were explored: (i) using only the concepts $x_{concept}$ as input, (ii) using the full latent space ($x_{latent}$ = [$x_{concept}$, $x_{style}$]) as input, and (iii) using only the style map $x_{style}$ as input. Also the influence of the linear kernel spatial size k for the Feature decoder block 314 on the evaluation scores was investigated, with different ranges, $k \in \{1, 3, 5, 7, 9\}$. To investigate the effect of the proposed training procedure, first comparison was made with a randomly initialized frozen model. The same random seed, dataset and number of linear-classifier optimization iterations were used throughout all scenarios.

Table 2 presents the linear evaluation results in terms of Dice Loss, which is equivalent to subtracting the Dice Score from 1. For both types of models (trained and randomly initialized) and across all $x_{input}$ setups, larger values of k result in lower test set losses. This is expected, as larger kernels capture more local information, and concepts cooperate locally to form larger anatomical structures. When $x_{input} := x_{latent}$ and the model is trained, the loss decreases only marginally when k exceeds 5 (i.e., the receptive field size used in the Feature Decoder block 314).

In all scenarios, ConceptVAE achieves lower test losses. For both models, the lowest losses occur when $x_{input} := x_{latent}$ (i.e., both concepts and styles are used for segmentation). When using only the concepts from the trained model, the losses are slightly higher but still significantly lower than when using only styles. Additionally, when $x_{input} := x_{style}$, the differences between the ConceptVAE and the random-init model are the smallest among all three input scenarios. This result brings further evidence that $x_{concept}$ contains semantic information useful for downstream tasks like segmentation, while $x_{style}$ focuses on local stylistic features. Moreover, there are virtually no differences in losses between using only $x_{concept}$ or only $x_{style}$ for the randomly initialised model, whereas these two scenarios yield substantial differences for ConceptVAE. This highlights the impact of our proposed unsupervised training framework on the model's ability to separate concepts from styles.

Table 2: Dice loss on the semantic segmentation test set when using $x_{concept}$ only, $x_{style}$ only, or $x_{concept}$ only, $x_{style}$ only, or $x_{concept}$ along with $x_{style}$. For each row, the lowest Dice losses are marked with bold.

|  | Kernel | Concept Only | Style Only | Concept & Style |
|---|---|---|---|---|
| Concept VAE | $1 \times 1$ | 0.5876 | 0.6641 | 0.4853 |
|  | $3 \times 3$ | 0.2268 | 0.4238 | 0.1741 |
|  | $5 \times 5$ | 0.1311 | 0.2586 | 0.1087 |
|  | $7 \times 7$ | 0.1013 | 0.1825 | 0.0938 |
|  | $9 \times 9$ | 0.0903 | 0.1520 | 0.0900 |
| Concept VAE Rand. | $1 \times 1$ | 0.6958 | 0.6942 | 0.6790 |
|  | $3 \times 3$ | 0.5413 | 0.5205 | 0.4655 |
|  | $5 \times 5$ | 0.3665 | 0.3504 | 0.2901 |

(continued)

Table 2: Dice loss on the semantic segmentation test set when using $x_{concept}$ only, $x_{style}$ only, or $x_{concept}$ only, $x_{style}$ only, or $x_{concept}$ along with $x_{style}$. For each row, the lowest Dice losses are marked with bold.

|  | Kernel | Concept Only | Style Only | Concept & Style |
|---|---|---|---|---|
| init. | $7 \times 7$ | 0.2465 | 0.2405 | 0.2016 |
|  | $9 \times 9$ | 0.1876 | 0.1990 | 0.1715 |
| Vicreg | $1 \times 1$ | 0.187 |  |  |

Also evaluation against the Vicreg baseline model was performed using a similar procedure, but only for the $1 \times 1$ sized convolutional kernel, and the outcomes are illustrated in Table 2. It is noted that ConceptVAE, using trained concepts and $5 \times 5$ windows or larger, achieves superior Dice metrics. This highlights the benefits of content-style disentanglement according to the inventive technique (briefly also: "the model") and the model's robustness against feature collapse.

To assess the proposed model's capability to detect OOD samples as a third downstream task, a test set comprising only parasternal long-axis (PLAX) views was employed. Unlike the test set used for the region-based instance retrieval, which includes only apical and short-axis acquisitions, this set is considered OOD because, although it contains echocardiographies, the views are different. The aim of this analysis is to determine whether the latent space features can differentiate between the two data distributions (i.e., apical and SAX versus PLAX views).

Most OOD methods are designed to work with supervised classification models, thus requiring explicit labeling either for in-domain classes or for flagging outlier samples. One method that does not require any labels and allows for fast log-likelihood evaluation with respect to the underlying data distribution is Normalising Flows (NFs). To this end, linear NFs were fitted solely on the frozen embeddings of in-distribution data (i.e., apical and SAX views) for both the proposed and baseline models.

The NF took the form of Equation (6), where $x$ represents an input derived from the latent space, $y$ is the transformed variable, and $A, b$ are trainable parameters.

$$y \ = \ Ax + \ b \ ,$$

$$\ln p\left(x\right) = \ln p_{prior}\left(y\right) + \ln|\det A| \qquad , \qquad\qquad (6)$$

$$p_{prior}(y) = \mathcal{N}\left(y \mid 0, I\right) .$$

For ConceptVAE, $x$ is formed by concatenating a $5 \times 5$ window of concept probabilities, excluding the style component. For the baseline model, $x$ is the feature embedding of a single location from the latent space feature grid. For all spatial locations corresponding to ultrasound cones within the latent space grid, and for all training data, the region descriptors $x$ were extracted and fed into the NF to maximize $\ln p(x)$ for in-distribution data. The same training data as for the semantic segmentation task was used to fit the NFs (i.e., only apical and SAX views). After the NFs converged, an image-level score was computed for each test sample by averaging the $\ln p(x)$ scores for all grid locations pertaining to the ultrasound cone.

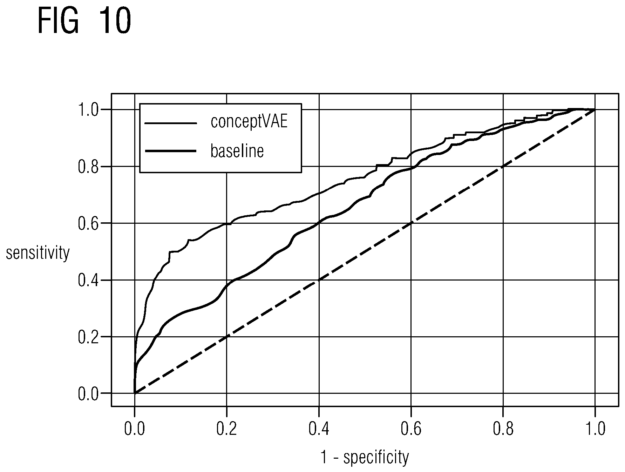

Two sets of image-level scores were computed, one for in-distribution apical and SAX views and one for OOD PLAX views. ROC curves were used to assess the score separability between the two sets using ConceptVAE and the Vicreg baseline, as shown in Fig. 10.

ConceptVAE has an area-under-curve of 0.753, being 10% larger than the baseline (with 0.655).

Fig. 10 show the receiver operating characteristic (ROC, as an example of a performance measure) curves comparison between ConceptVAE and the Vicreg baseline model, for distinguishing in-distribution echocardiographic views from OOD PLAX ones. ConceptVAE has an AuROC score of 0.753, while the Vicreg baseline has an AuROC of 0.655.

In contrast to the ConceptVAE according to the technique disclosed herein, the baseline model had access to PLAX data during its development (a vast collection of many echocardiography types was used to pretrain the baseline model, following common practices for classical self-supervised pretraining regarding dataset sizes and variability, therefore the PLAX view is not OOD for the baseline model). Also, the contrastive objective used for developing the baseline model should promote feature clustering w.r.t. data sub-groups (e.g., anatomical views). Despite this fact, ConceptVAE produces local embeddings that are more separable between echocardiographic views (even near-OOD ones), again indicating a reduction of feature collapse due to the content-style disentanglement. This behavior of embeddings separability even for near-OOD data does not usually manifest for regular deep-neural networks.

To further evaluate the generalization capability of ConceptVAE, as a further downstream task, an aim is to detect latent space grid locations corresponding to the aortic valve (AV) region in views not used during pre-training (i.e., PLAX). Similarly to the semantic segmentation task, for the AV detection task, a linear convolutional layer is trained on top of frozen embeddings to perform a proxy object detection task. Each testing sample has a bounding box annotation around the AV along with a label indicating if it is open or closed (depending on the cardiac phase depicted in the test image). The bounding boxes were downsized to the output stride of the latent space and used an overlap threshold t to determine the objectness of each latent space grid location, i.e., if the down-sampled bounding box overlaps a grid location with a ratio larger than $t$, then that grid location objectness is set as 1, otherwise 0. Moreover, for each object grid location the newly added convolutional layer also predicts the AV state (open or closed).

For ConceptVAE, the input to the linear layer is a $5 \times 5$ window of both concept probabilities and associated styles for the concepts having the highest probability. The output consists of 3 channels, one for classifying objectness and the other two for classifying the AV state. For the baseline Vicreg model, the setup is similar, but the input is the feature vector of a $1 \times 1$ latent space grid location. Balanced binary cross-entropy losses are employed to train both objectives (i.e., detection and labeling).

The results are illustrated in Table 3. The mAP scores are close (with the baseline slightly better by 1.6% mAP), while the objectiveness AP is much larger for the technique disclosed herein (+12%). This is because the inventive technique does a better job in locating Aortic Valve grid positions, but somewhat lags in correctly classifying the AV state for the detected AV locations. It is hypothesized that locating the AV can be done by analyzing concepts (e.g., exploiting a linear separability of concept probabilities w.r.t. AV presence) while the AV state can be inferred from the style component of the latent space. To test this, a new linear layer was trained only on the concept components of the latent space and a severe degradation in label classification performance was observed while retaining the objectness classification performance. The previous section revealed that the detected concepts on the near-OoD PLAX views are still descriptive of the image's semantics; however, the style component may not fully capture all relevant fine details, since the proposed model was not trained on PLAX views as opposed to the baseline model.

Table 3. Mean average precision scores for object detection on PLAX views.

| Metric | Model | |
| --- | --- | --- |
| | ConceptVAE | Baseline |
| "open-AV" class AP | 0.337 | 0.297 |
| "closed-AV" class AP | 0.386 | 0.459 |
| mean AP | 0.362 | 0.378 |
| objectness AP | 0.786 | 0.665 |

Further, it was explored how style information can be used to generate synthetic data. Such data can be valuable for creating inputs conditioned by patient attributes, such as generating images with more textured walls. To achieve this, the known range of $x_{style}$ was leveraged (since the constraint $\mathcal{L}_{style}$ is enforced during training), and style-based image generation was investigated. This involved adding Gaussian noise at various levels $\beta$ as described in Equation (7):

$$n \sim \mathcal{N}(0, I) \,,$$

$$x^*_{style} = \frac{x_{style} + \beta n}{\sqrt{1 + \beta^2}} \,, \qquad (7)$$

where $\beta$ controls the amount of noise injected into $x^*_{style}$.

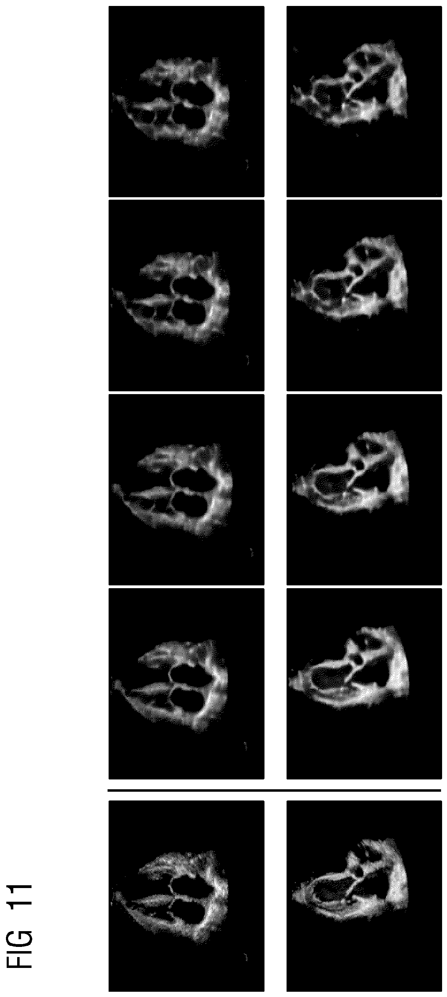

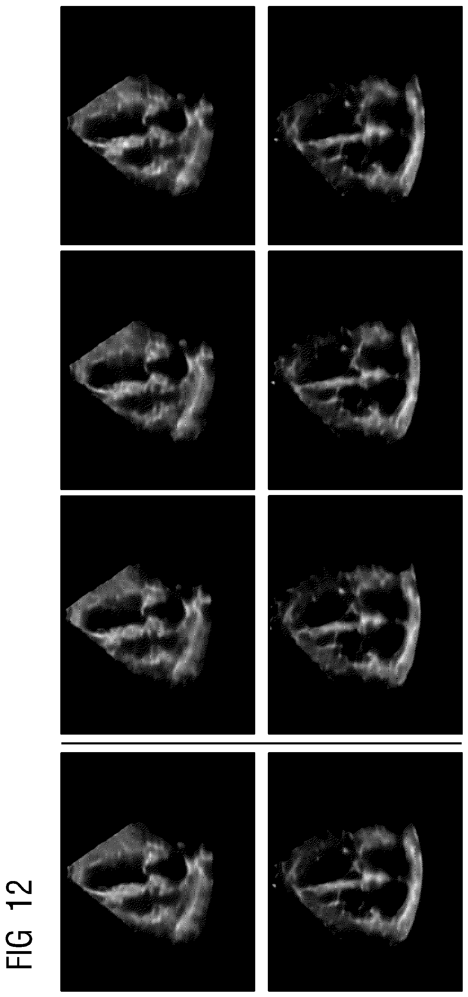

The image was then reconstructed using these style attributes. Randomly sampled reconstructions w.r.t. multiple $\beta$ (reusing the same sampled n) are illustrated in Fig. 11, while Fig. 12 illustrates reconstructions with multiple noise samplings $n_k \sim \mathcal{N}(0, I)$ and fixed $\beta = 0.3$. It is observed that even with relatively high $\beta$ values, the reconstructions closely resemble the unaltered concepts, while the image textures are modified (with minimal changes to anatomical structures in terms of their shape or placement). This leads to the following observations:

The model uses $x_{concept}$ to decode semantic content, such as anatomical structures like chamber walls, blood pools, and valves, while $x_{style}$ is used to particularize local textures, shadows and speckles.

With ConceptVAE, synthetic data can be generated by modifying only textures and speckles while retaining anatomical structures. This allows for the generation of novel samples that can serve as style augmentations without modifying the content, potentially enhancing the training performance of dense downstream models, such as those used for segmentation.

In Fig. 11, original images (left) are displayed alongside reconstructions using $x^*_{style}$ with increasing levels of injected noise, $\beta$. From the second column to the right, $\beta$ values are 0 (unaltered reconstruction), 0.2, 0.4 and 0.6, respectively.

In Fig. 12, reconstructed images with unaltered $x_{style}$ (left) alongside three reconstructions with constant noise level $\beta = 0.3$ are shown. Each noisy reconstruction uses different noise, $n \sim \mathcal{N}(0, I)$, as described in Equation (7).

The samples generated with ConceptVAE remain within the original data distribution, and thus can serve as a more calibrated augmentation method. In contrast, classical transformations such as rotations and blurring may generate data points with appearances not observed in the initial distribution (e.g., unnatural rotations or texture changes). Ultrasound medical imaging inherently introduces noise in video acquisitions in the form of pixel speckles. ConceptVAE simulates the effect of different realizations of echocardiography-specific noise, producing images that reflect this variability. Given the large variability between acquisitions and patients in ultrasound imaging, the inventive technique can potentially improve the robustness of the models on downstream tasks.

ConceptVAE is an example of the generic inventive technique (and/or SSL framework) designed to learn disentangled representations, namely for the example of 2D cardiac ultrasound images. This technique involves converting input embeddings into a set of discrete concepts and associated continuous styles. Through multiple qualitative and quantitative analyses, it was demonstrated that ConceptVAE captures anatomical information within concepts vectors and local textures within the style vectors, thereby achieving disentanglement. For example, by qualitatively analyzing the concept maps, it was observed that the technique is able to specialize certain concepts to independent anatomical structures such as blood pools or septum walls. These properties prove beneficial for several downstream applications, including region-based instance retrieval, object detection, and synthetic data generation. Specifically, empirical evidence was provided that ConceptVAE outperforms conventional SSL methods like Vicreg in region-based instance retrieval, OOD detection, semantic segmentation, and object detection. Moreover, the technique shows promising results in generating synthetic data samples that reflect the original data distribution and preserve anatomical concepts while varying styles.

The data used for the empirical experiments are courtesy of Princeton Radiology and Zwanger Pesiri.

Independently of the grammatical term usage, individuals with male, female or other gender identities are included within the term.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

Cited Prior Art

[1] Shih-Cheng Huang et al., Self-supervised learning for medical image classification: a systematic review and implementation guidelines, njp Digital Medicine (2023)6:74

[2] Taleb, Aiham, et al. "3d self-supervised methods for medical imaging." Advances in neural information processing systems 33 (2020): 18158-18172.

[3] Wang, Yiqing, et al. "Swinmm: masked multi-view with swin transformers for 3d medical image segmentation." International conference on medical image computing and computer-assisted intervention. Cham: Springer Nature Switzerland, 2023.

[4] Jingyang Lin et al., CT-GLIP: 3D Grounded Language-Image Pretraining with CT Scans and Radiology Reports for Full-Body Scenarios, arXiv:2404.15272v3 [cs.CV]

**Claims**

**1.** Computer-implemented method (100) for pre-training a principal encoder and a concept head, in particular for performing a downstream perception task, comprising the method steps of:

- Receiving (S102), at an input layer of a principal encoder, a medical image;
- Processing (S104), by the principal encoder, the medical image for obtaining a principal latent representation of the received (S102) medical image;
- Providing (S106) the principal latent representation to a concept head;
- Obtaining (S108), by the concept head, a first vector of discretized anatomical concepts based on the principal latent representation;
- Providing (S110) the principal latent representation and the obtained (S108) first vector of discretized anatomical concepts to a style head;
- Obtaining (S112), by the style head, a further first vector of continuous styles per discretized anatomical concept in the medical image based on the first vector of discretized anatomical concepts and on the principal latent representation;
- Determining (S114-C), by an auxiliary feature decoder, a first auxiliary latent representation based on the obtained first vector of discretized anatomical concepts;
- Performing (S116-C), by an auxiliary image decoder, a first reconstruction of the medical image based on the determined first auxiliary latent representation; and
- Pre-training (S132) the principal encoder and the concept head, wherein the pre-training (S132) is based on optimizing a loss function comprising a reconstruction loss between the received (S102) medical image and the first reconstruction of the medical image.

2. Method (100) according to claim 1, further comprising the steps of:

- Determining (S114-CS), by the auxiliary feature decoder, a further first auxiliary latent representation based on the obtained first vector of discretized anatomical concepts and the further first vector of continuous styles per discretized anatomical concept;
- Performing (S114-CS), by the auxiliary image decoder, a second reconstruction of the medical image based on the determined further first auxiliary latent representation;

wherein the pre-training (S132) is further based on optimizing a loss function comprising a reconstruction loss between the received (S102) medical image and the second reconstruction of the medical image.

3. Method (100) according to claim 1 or 2, further comprising the steps of:

- Receiving (S120), at an input layer of an auxiliary encoder, an augmented (S119) version of the medical image, in particular in parallel to the receiving (S102) of the medical image at the input layer of the principal encoder;
- Processing (S122), by the auxiliary encoder, the augmented medical image for obtaining a second auxiliary latent representation of the augmented medical image;
- Providing (S124) the second auxiliary latent representation to the concept head; and
- Obtaining (S126), by the concept head, a second vector of discretized anatomical concepts based on the second auxiliary latent representation;

wherein the pre-training (S132) is further based on optimizing the loss function comprising a concept consistency loss between the first vector of discretized anatomical concepts and the second vector of discretized anatomical concepts.

4. Computer-implemented method (200) for pre-training a principal encoder and a concept head, in particular for performing a downstream perception task, comprising the method steps of:

- Receiving (S202), at an input layer of the principal encoder, a medical image;
- Processing (S204), by the principal encoder, the medical image for obtaining a principal latent representation of the received (S202) medical image;
- Providing (S206) the principal latent representation to a concept head;
- Obtaining (S208), by the concept head, a first vector of discretized anatomical concepts based on the principal latent representation;
- Providing (S210) the principal latent representation and the obtained (S108) first vector of discretized anatomical concepts to a style head;
- Obtaining (S212), by the style head, a further first vector of continuous styles associated with the first vector of discretized anatomical concepts and the principal latent representation;

- Receiving (S220), at an input layer of an auxiliary encoder, an augmented (S219) version of the medical image, in particular in parallel to the receiving (S202) at the input layer of the principal encoder;
- Processing (S222), by the auxiliary encoder, the augmented (S114) medical image for obtaining a second auxiliary latent representation of the augmented (S114) medical image;
- Providing (S224) the second auxiliary latent representation to the concept head;
- Obtaining (S126), by the concept head, a second vector of discretized anatomical concepts based on the second auxiliary latent representation; and
- Pre-training (S232) the principal encoder and the concept head, wherein the pre-training (S232) is based on optimizing a loss function comprising a concept consistency loss between the first vector of discretized anatomical concepts and the second vector of discretized anatomical concepts.

5. Method (100; 200) according to any one of claims 1,2, or 4, further comprising the step of:

- Constructing (S118; S218) a grid of discretized anatomical concepts based on the first vector of discretized anatomical concepts, wherein the grid is constructed (S118; S218) by assigning each entry of the first vector to its associated point on a lattice covering the area or volume of the medical image;

wherein the pre-training (S132; S232) is further based on optimizing the loss function comprising a concept cluster loss and/or concept prior loss of the grid of discretized anatomical concepts.

6. Method (100; 200) according to any one of the preceding claims, wherein the style head is pre-trained based on a style covariance loss, optionally wherein the style covariance loss comprises a constraint on unit covariance and zero mean along a grid dimensions.

7. Method (100; 200) according to any of the preceding claims, wherein the principal encoder and a principal image decoder are comprised in a principal encoder-decoder pair, the method (100; 200) further comprising the steps of:

- Receiving (S128-P; S228-P) the principal latent representation at the principal image decoder; and
- Outputting (S130-P; S230-P), by the principal image decoder, a reconstruction of the medical image;

wherein pre-training (S132; S232) the principal encoder, and optionally the principal image decoder, is further based on optimizing the loss function comprising a reconstruction loss between the medical image and the reconstruction output by the principal decoder.

8. Method (100; 200) according to any one of the preceding claims, wherein an auxiliary encoder and an auxiliary image decoder are comprised in an auxiliary encoder-decoder pair, the method (100; 200) further comprising the steps of:

- Receiving (S128-A; S228-A), at the auxiliary image decoder, a second auxiliary latent representation output by the auxiliary encoder based on an augmented version of the medical image; and
- Outputting (S130-A; S230-A), by the auxiliary image decoder, a reconstruction of the augmented medical image;

wherein the auxiliary encoder-decoder pair is pre-trained (S132; S232) based on minimizing a reconstruction loss between the augmented medical image and the reconstruction output by the auxiliary image decoder.

9. Method (100; 200) according to the directly preceding claim, wherein pre-training the principal encoder, the concept head, the style head, and optionally the principal image decoder, comprises pre-training the auxiliary encoder-decoder pair

- by using the concept head and the style head, for modifying parameters and/or weights of the auxiliary encoder-decoder pair; and/or
- using the auxiliary encoder-decoder pair for, in particular directly, modifying parameters and/or weights of the concept head and the style head, and in particular indirectly modifying parameters and/or weights of the principal encoder.

10. Method (100; 200) according to any of the preceding claims, wherein augmenting (S114) the medical image comprises at least one of cropping, zooming, blurring, color jittering, adding noise, masking, translating, rotating, spatially shifting, shearing, and/or gamma contrast changing the medical image.

**11.** Method (100; 200) according to any of the preceding claims, wherein

- the discretized anatomical concepts comprise organs, anatomical structures, and/or their constituent parts; and/or optionally wherein
- the continuous styles comprise low-level information in relation to the associated discretized anatomical concepts, such as texture data, tissue type data, and/or any further detailed features in relation to the concept to which the style is associated.

**12.** Method (100; 200) according to any of the preceding claims, wherein a concept is attributed to a minimal size of a set of adjacent pixels or voxels.

**13.** Method (100; 200) according to any of the preceding claims, wherein a downstream perception task to be performed on the medical image is selected from at least one of:

- an information retrieval;
- a reconstruction;
- an object classification;
- an object detection;
- a semantic segmentation;
- a pattern recognition;
- a disease identification;
- a region-based instance retrieval;
- an Out-of-Distribution, OOD, detection;
- a classification if a valve is open or closed; and
- synthetic data generation.

**14.** Use of a principal encoder, concept head and style head pre-trained according to the method according to any one of the preceding claims in combination with a downstream perception task-specific head for performing the downstream perception task.

**15.** A pre-training network architecture (300) for pre-training a principal encoder and a concept head, in particular for performing a downstream perception task, comprising:

- A principal encoder (302), which is configured for rreceiving, at an input layer, a medical image and is further configured for processing the medical image for obtaining a principal latent representation of the received medical image;
- A concept head (306), which is configured for receiving the principal latent representation and is further configured for obtaining a first vector of discretized anatomical concepts based on the principal latent representation;
- A style head (310), which is configured for receiving the principal latent representation and the obtained first vector of discretized anatomical concepts to a style head and further configured for obtaining a further first vector of continuous styles per discretized anatomical concept in the medical image based on the first vector and on the principal latent representation;
- An auxiliary feature decoder (314), which is configured for determining a first auxiliary latent representation based on the obtained first vector of discretized anatomical concepts;
- An auxiliary image decoder (316), which is configured for performing a first reconstruction of the medical image based on the determined first auxiliary latent representation; and
- A loss function (332), which is configured for pre-training the principal encoder and the concept head, wherein the pre-training is based on optimizing a loss function comprising a reconstruction loss between the received medical image and the first reconstruction of the medical image.

**16.** A downstream perception task network architecture (400), comprising:

- a principal encoder (302);
- a concept head (306);
- a style head (310); and
- a downstream perception task-specific head (440);

wherein the principal encoder (302), the concept head (306) and the style head (310) have been pre-trained using the method (100; 200) according to any of the preceding method claims.

FIG 1

EP 4 712 046 A1

EP 4 712 046 A1

FIG 2

200

S202 → S204 → S206 → S208 → S210 → S212

S208 → S218

S212 → S214-C, S214-CS

S214-C → S216-C

S214-CS → S216-CS

S219 → S220 → S222 → S224 → S226

S222 → S228-A

S226 → S232

S228-P → S230-P

S228-A → S230-A

S216-C, S216-CS, S230-P, S230-A → S232

EP 4 712 046 A1

## FIG 3

300

336

302    314    320

306    316    328

310    318    332

338

334

## FIG 4

400

336

302

306

310

440

434

338

FIG 5

EP 4 712 046 A1

# FIG 6

502

506

508

602

4x output stride

302

328

s.g. =

504

514-C

16x output stride

306

606

320

510

306'

310

612

610

608

320

314

314

320

508-B

4x output stride

604

316

316

604

602

602

512-CS

512-C

FIG 7A

FIG 7B

FIG 7C

FIG 8A

FIG 8B

FIG 8C

# FIG 9

| A2C LA ES | *A2C LA ED | A2C ED | A3C ES |
| A4C RV ES | A4C RV ES | *A4C RV ED | A4C ES |
| A2C ED | A2C ED | *A2C ES | A2C ED |
| A3C ED | *A3C ES | A3C ED | A3C ES |
| SAX ES | SAX ES | SAX ED | SX ES |
| A4C ES | *A4C ED | A4C ES | A4C ES |

FIG 10

FIG 11

FIG 12

EP 4 712 046 A1

Europäisches
Patentamt

European
Patent Office

Office européen
des brevets

## EUROPEAN SEARCH REPORT

**Application Number**

EP 25 18 2637

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | CIUSDEL COSTIN F. ET AL: "ConceptVAE: Self-Supervised Fine-Grained Concept Disentanglement from 2D Echocardiographies", APPLIED SCIENCES, vol. 15, no. 3, 30 January 2025 (2025-01-30), page 1415, XP093320198, Basel ISSN: 2076-3417, DOI: 10.3390/app15031415 * abstract * * Section 3 * | 1-16 | INV. G06V10/82 G06N3/0455 |
| A | CHARTSIAS AGISILAOS ET AL: "Disentangled representation learning in cardiac image analysis", MEDICAL IMAGE ANALYSIS, OXFORD UNIVERSITY PRESS, OXOFRD, GB, vol. 58, 18 July 2019 (2019-07-18), XP085878769, ISSN: 1361-8415, DOI: 10.1016/J.MEDIA.2019.101535 [retrieved on 2019-07-18] * abstract * * Section 2.2 * * Section 3 * | 1-16 | |

| | | |
|---|---|
| **TECHNICAL FIELDS SEARCHED (IPC)** | |
| G06V | |
| G06N | |

The present search report has been drawn up for all claims

| Place of search | Date of completion of the search | Examiner |
|---|---|---|
| The Hague | 30 September 2025 | Boltz, Sylvain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P4C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHIH-CHENG HUANG et al.** Self-supervised learning for medical image classification: a systematic review and implementation guidelines. *njp Digital Medicine*, 2023, vol. 6, 74
- **TALEB, AIHAM et al.** 3d self-supervised methods for medical imaging. *Advances in neural information processing systems*, 2020, vol. 33, 18158-18172

- Swinmm: masked multi-view with swin transformers for 3d medical image segmentation. **WANG, YIQING et al.** International conference on medical image computing and computer-assisted intervention. Cham: Springer Nature Switzerland, 2023
- **JINGYANG LIN et al.** CT-GLIP: 3D Grounded Language-Image Pretraining with CT Scans and Radiology Reports for Full-Body Scenarios. *arXiv:2404.15272v3*